# EUROPEAN PATENT APPLICATION

(11) **EP 4 473 974 A1**
(43) Date of publication of application: **11.12.2024**
(21) Application number: 23382552.0
(22) Date of filing: 07.06.2023
(51) Int. Cl.: A61K 38/16, A61K 31/519, A61K 45/06, A61P 35/00

(54) **OMOMYC AND KRAS INHIBITORS COMBINATION THERAPY FOR THE TREATMENT OF CANCER**

(71) Applicant: Peptomyc, S.L., 08035 Barcelona (ES); Fundació Privada Institut d'Investigació Oncològica de Vall Hebron, 08035 Barcelona (ES); Institució Catalana de Recerca i Estudis Avançats, 08010 Barcelona (ES)
(72) Inventor: CASACUBERTA SERRA, Sílvia, E-08035 Barcelona (ES); BEAULIEU, Marie-Eve, E-08035 Barcelona (ES); SOUCEK, Laura, E-08010 Barcelona (ES); GONZÁLEZ LARREATEGUI, Íñigo, E-08035 Barcelona (ES)
(74) Representative: ABG Intellectual Property Law, S.L.

(57) **Abstract**

The invention relates to a combination of a KRAS inhibitor with Omomyc, a functionally equivalent variant thereof, a conjugate comprising Omomyc or said functionally equivalent variant, a polynucleotide encoding said polypeptides, a vector comprising said polynucleotide and a cell capable of secreting the polypeptide or the conjugate. The invention also relates to pharmaceutical compositions containing the combination of the invention and to their medical uses, particularly their uses in the treatment of cancer.

## Description

### FIELD OF THE INVENTION

The present invention relates to the field of cancer and, more particularly, to a combination comprising Kirsten rat sarcoma 2 viral oncogene homolog (KRAS) inhibitors and Omomyc and its use in medicine, more particularly in the prevention and/or treatment of cancer.

### BACKGROUND OF THE INVENTION

Cancer is a leading cause of death worldwide, accounting for nearly 10 million deaths in 2020. It is a large subset of diseases characterized by the uncontrollable growth of abnormal cells.

RAS is a well-known proto-oncogene and its gain-of-function mutations occur in approximately 30% of all human cancers. The most frequently mutated RAS isoform is KRAS, which constitutes 86% of RAS mutations. KRAS-4B is the dominant isoform in human cancers, and it is present in approximately 90% of pancreatic cancers, 30% to 40% of colon cancers, and 15% to 20% of lung cancers, mostly non-small-cell lung cancer (NSCLC). It is also present in biliary tract malignancies, endometrial cancer, cervical cancer, bladder cancer, liver cancer, myeloid leukemia and breast cancer.

The most frequent mutations in the KRAS gene are primarily at codons 12, 13, or 61. Over 80% of mutations occur at codon 12, normally occupied by a glycine residue. KRAS mutations also occur in codons 63, 117, 119, and 146 but with less frequency.

A huge number of KRAS inhibitors are now under development to treat KRAS mutant cancer either directly towards mutant KRAS or against the crucial steps required for KRAS activation.

KRAS-G12C oncoprotein is one of the three most common KRAS mutants in cancer, present in 10-20% of all KRAS G12C mutations and approximately 50% of KRAS-driven lung adenocarcinomas. Direct KRAS-G12C inhibitors such as sotorasib and adagrasib selectively target KRAS G12C mutations and are used to treat patients with advanced non-small cell lung cancer (NSCLC) carrying KRAS-G12C mutations. Unfortunately, many patients do not respond to KRAS-G12C inhibitor therapy due to intrinsic or acquired resistance.

Beside the KRAS-glycine-to-cysteine mutation at codon 12 (KRASG12C), another very common and prevalent mutation in the KRAS oncogene is the G12D, glycine-to-aspartic acid mutation at codon 12. G12D is most commonly see in pancreatic ductal adenocarcinoma, a dismal disease with an average 5-year survival rate of less than 10% due to difficult early diagnosis and lack of effective treatment. Additional efforts are made to optimize the activity of KRAS-G12D inhibitors and overcome their resistance.

Another RAS pathway-targeted treatments are the so-called pan-KRAS inhibitors that do not discriminate between KRAS mutants and can thus be used against non-G12C mutants. Pan-KRAS inhibitors are able to target the KRAS pathway regardless of the specific KRAS mutation. These include both direct and indirect pan-KRAS drugs such as those that interfere with KRAS nucleotide exchange and activation by inhibiting SHP2 or SOS1. SOS1 is a key guanine exchange factor (GEF) for KRAS that binds and activates GDP-bound RAS family proteins at its catalytic binding site and in this way promotes exchange of GDP for GTP. Particular attention has been drawn to pan-KRAS SOS1 inhibitors such as BI-3406 and BAY-293. These molecules bind to SOS1, reducing the formation of active GTP-loaded KRAS. However, BAY-293 inhibited cancer cell proliferation with weak potency.

Despite recent advances in clinically available small molecules that target KRAS, only around 30% of the patients respond and benefit from these therapies. Thus, cancer patients bearing KRAS mutations still represent a highly unmet medical need.

Therefore, there is still a need in the art to develop novel and improved therapeutic approaches for the treatment of cancer that can overcome resistance to KRAS inhibitors increasing its potency and/or reducing their adverse effects.

### BRIEF SUMMARY OF THE INVENTION

In a first aspect, the invention relates to a combination comprising:
i) a first component selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b);
   and
ii) a second component that is a KRAS inhibitor.

In a second aspect, the invention relates to a pharmaceutical composition comprising a pharmaceutically effective amount of a combination according to the invention and a pharmaceutically acceptable excipient.

In a third aspect, the invention relates to a combination according to the invention or a pharmaceutical composition according to the invention for use in medicine.

In a fourth aspect, the invention relates to a combination according to the invention or a pharmaceutical composition according to the invention for use in the prevention and/or treatment of cancer.

### DESCRIPTION OF THE FIGURES

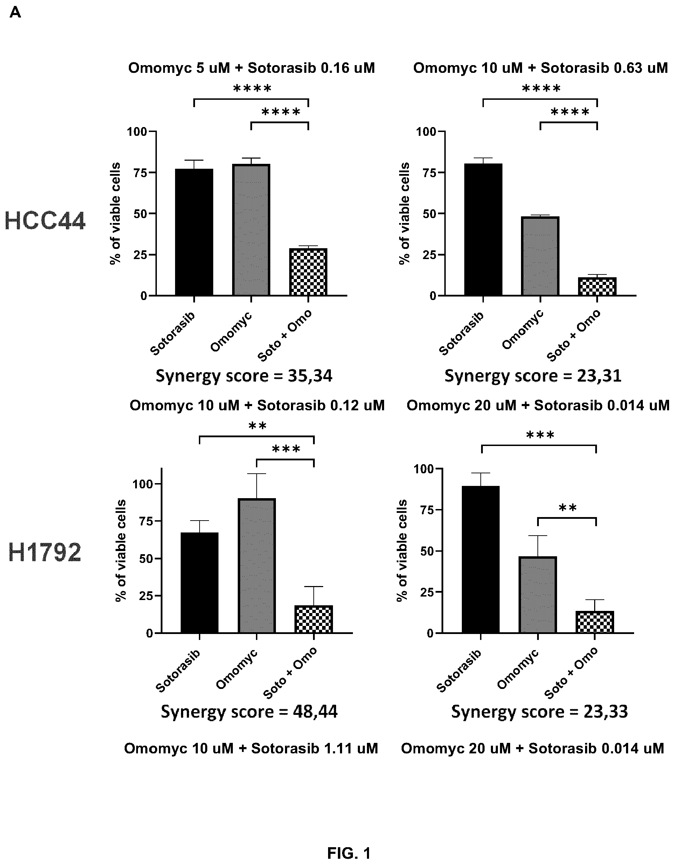

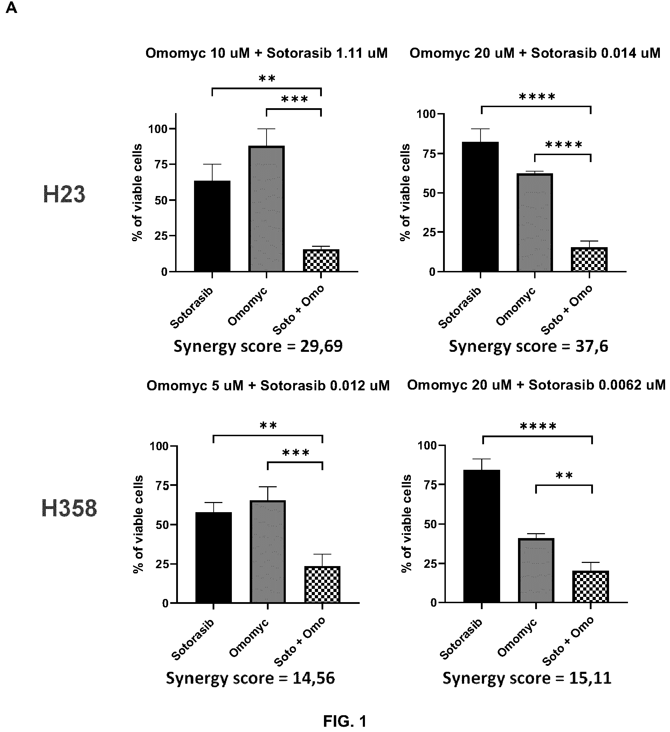

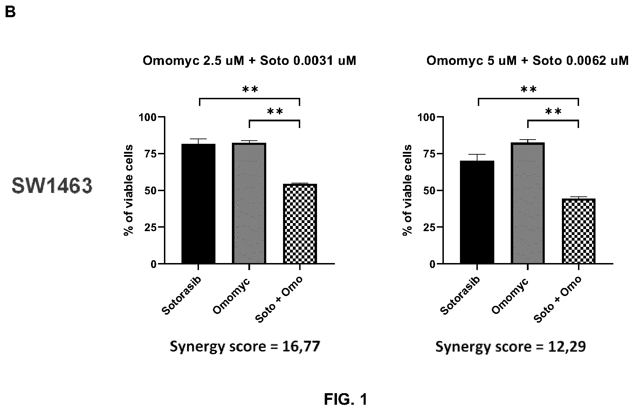

**Figure 1****. Synergy with Sotorasib (a KRAS G12C inhibitor) in KRASG12C-mutant NSCLC and colorectal (CRC) cell lines *in vitro.*** Results show that Omomyc synergizes with Sotorasib in all cell lines in many different concentrations, significantly reducing the number of viable cells. **A.** Bar graphs showing percentage of viable cells of NSCLC cell lines treated with Omomyc and Sotorasib in representative synergistic concentrations. Their associated synergy score is shown below each graph. **B.** Bar graphs showing percentage of viable cells of a CRC cell line treated with Omomyc and Sotorasib in representative synergistic concentrations. Their associated synergy score is shown below each graph. Synergy scores were calculated using the SynergyFinder software. Mean and Standard Deviation is shown. ** p < 0.01; *** p < 0.001; **** p < 0.0001

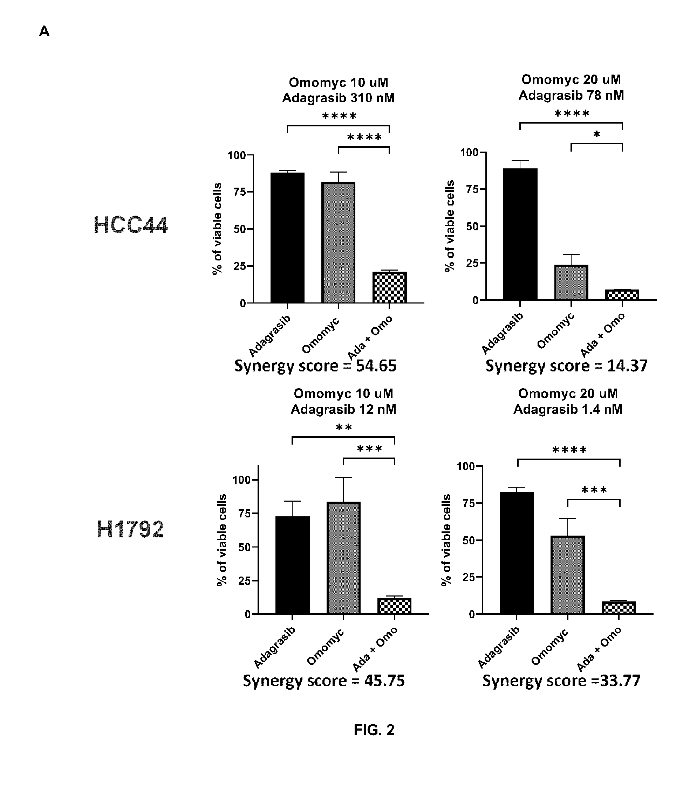

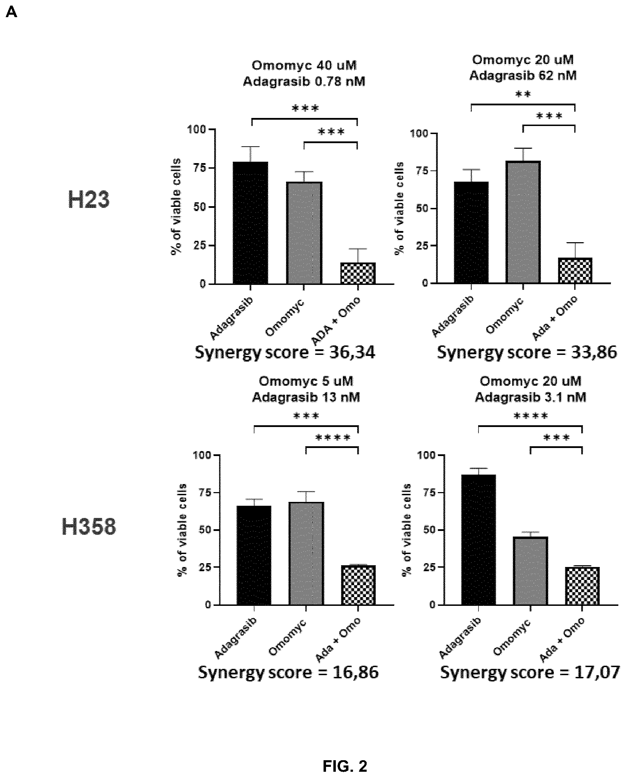

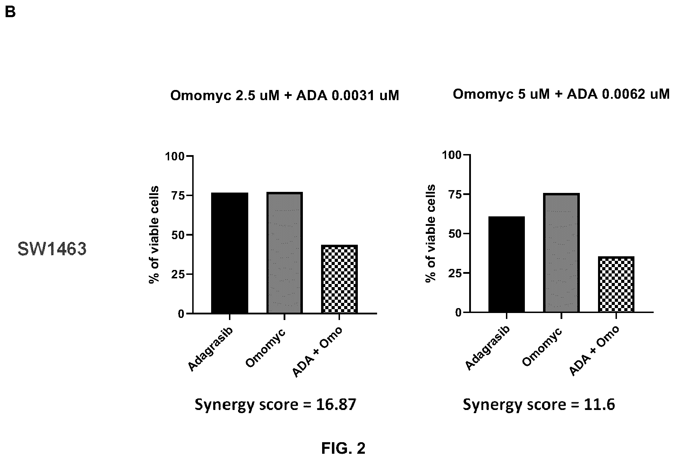

**Figure 2****. Synergy with Adagrasib (a KRAS G12C inhibitor) in KRASG12C-mutant NSCLC and CRC cell lines *in vitro.*** Results show that Omomyc synergizes with Adagrasib in all cell lines in many different concentrations, significantly reducing the number of viable cells. **A.** Bar graphs showing percentage of viable cells of NSCLC cell lines treated with Omomyc and Adagrasib in representative synergistic concentrations. Their associated synergy score is shown below each graph. **B.** Bar graphs showing percentage of viable cells of a CRC cell line treated with Omomyc and Adagrasib in representative synergistic concentrations. Their associated synergy score is shown below each graph. Synergy scores were calculated using the SynergyFinder software. Mean and Standard Deviation is shown. * p < 0.05; ** p < 0.01; *** p < 0.001; **** p < 0.0001

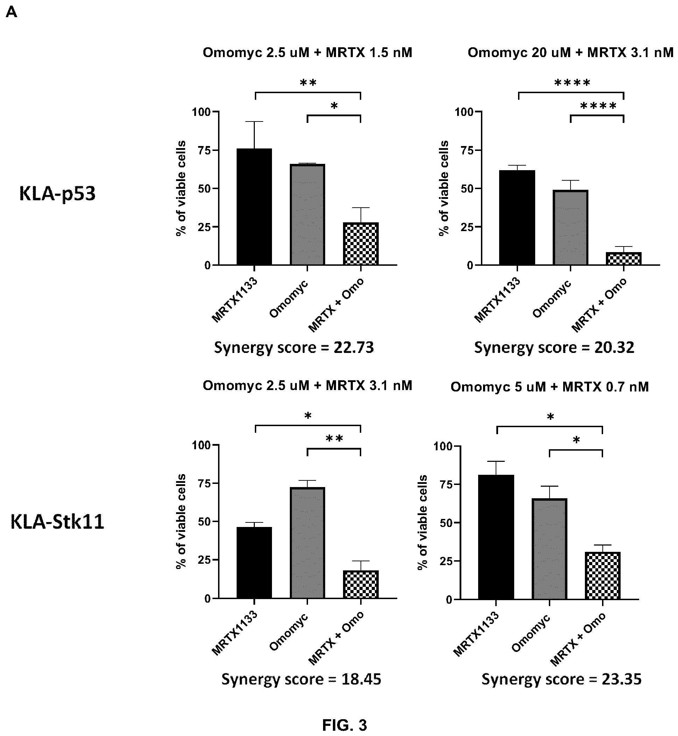

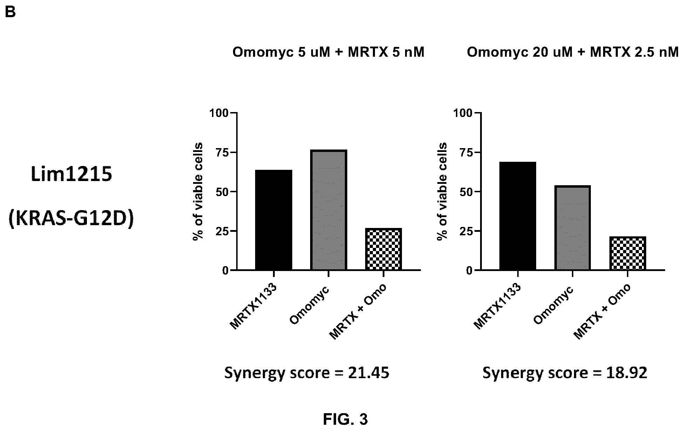

**Figure 3****. Synergy with MRTX1133 (a KRAS G12D inhibitor) in KRASG12D-mutant NSCLC and CRC cell lines *in vitro.*** Results show that Omomyc synergizes with MRTX1133 in all cell lines in many different concentrations, significantly reducing the number of viable cells. **A.** Bar graphs showing percentage of viable cells of NSCLC cell lines treated with Omomyc and MRTX1133 in representative synergistic concentrations. Their associated synergy score is shown below each graph. **B.** Bar graphs showing percentage of viable cells of a CRC cell line treated with Omomyc and MRTX1133 in representative synergistic concentrations. Their associated synergy score is shown below each graph. Synergy scores were calculated using the SynergyFinder software. Mean and Standard Deviation is shown. * p < 0.05; ** p < 0.01; **** p < 0.0001

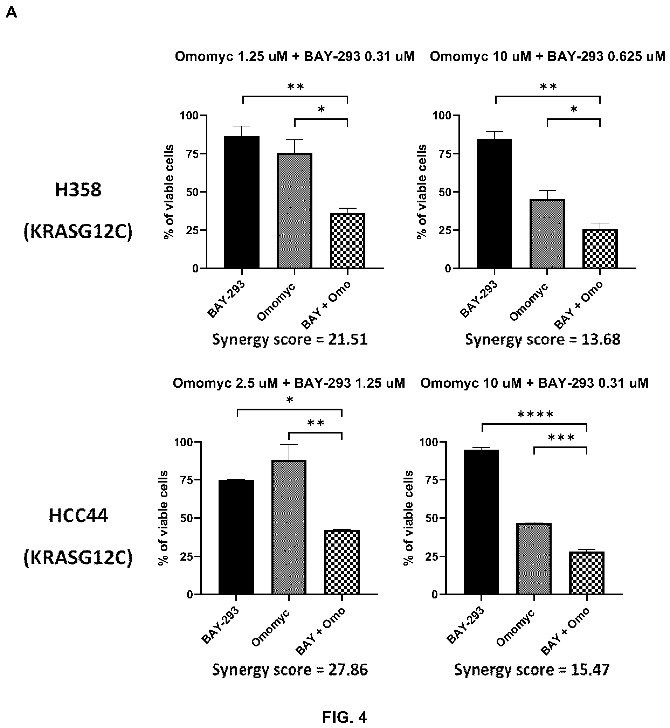

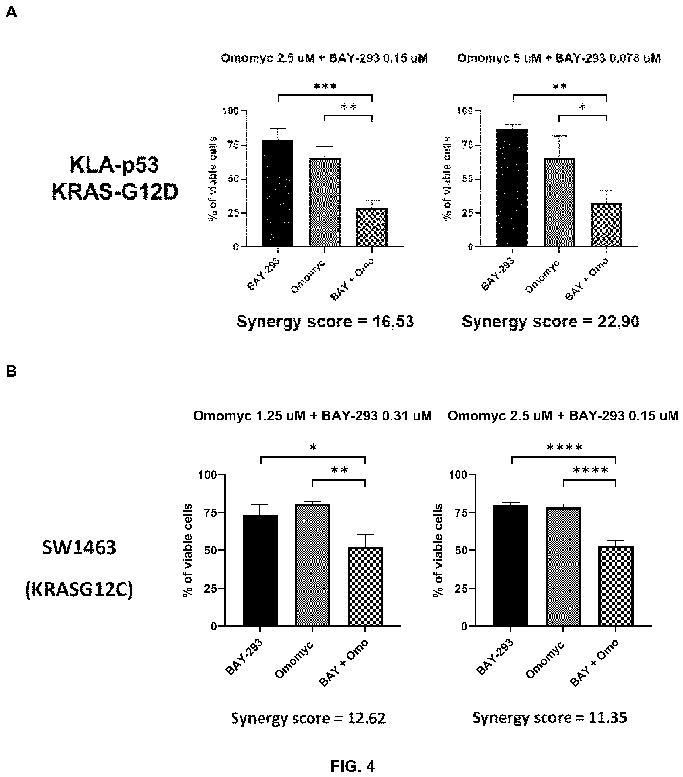

**Figure 4****. Synergy with BAY-293 [a pan-KRAS (SOS1) inhibitor] in KRAS-mutant NSCLC and CRC cell lines *in vitro.*** Results show that Omomyc synergizes with BAY-293 in all cell lines, regardless of their KRAS mutation, in many different concentrations, significantly reducing the number of viable cells. **A.** Bar graphs showing percentage of viable cells of NSCLC cell lines treated with Omomyc and BAY-293 in representative synergistic concentrations. Their associated synergy score is shown below each graph. **B.** Bar graphs showing percentage of viable cells of a CRC cell line treated with Omomyc and BAY-293 in representative synergistic concentrations. Their associated synergy score is shown below each graph. Synergy scores were calculated using the SynergyFinder software. Mean and Standard Deviation is shown. * p < 0.05; ** p < 0.01; *** p < 0.001; **** p < 0.0001

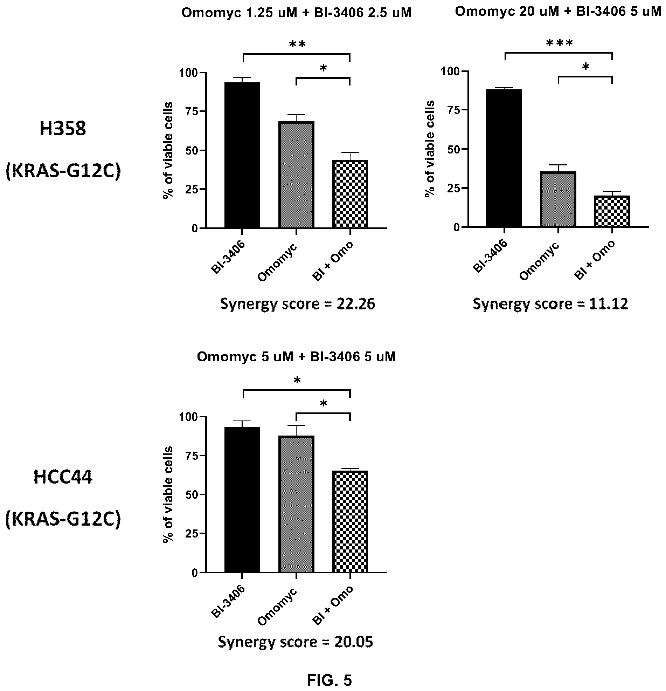

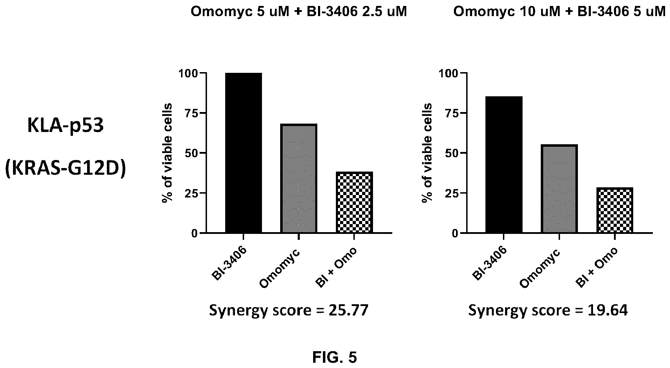

**Figure 5****. Synergy with BI-3406 [a pan-KRAS (SOS1) inhibitor] in KRAS-mutant NSCLC cell lines *in vitro.*** Results show that Omomyc synergizes with BI-3406 in all cell lines, regardless of their KRAS mutation, in many different concentrations, significantly reducing the number of viable cells. Bar graphs showing percentage of viable cells of NSCLC cell lines treated with Omomyc and BI-3406 in representative synergistic concentrations. Their associated synergy score is shown below each graph. Synergy scores were calculated using the SynergyFinder software. Mean and Standard Deviation is shown. * p < 0.05; ** p < 0.01; *** p < 0.001

### DETAILED DESCRIPTION OF THE INVENTION

The present invention relates to the provision of new therapeutic combinations for the prevention and treatment of cancer.

Unless otherwise defined, all technical terms used herein have the same meaning as commonly understood by one of ordinary skill in the art to which this invention belongs.

All the embodiments disclosed in relation to an aspect of the invention are applicable to the other aspects.

### Combinations and pharmaceutical compositions of the invention

The definitions provided herewith and in every other aspect of the invention are equally applicable to the whole invention.

The authors of the present invention have surprisingly found that the combination of Omomyc and a KRAS inhibitor has a synergistic effect in treating cancers. The authors have shown synergistic effects of a combination of Omomyc with different KRAS inhibitors that target the most common specific mutations of the KRAS oncogene (G12C and G12D) but also pan-KRAS inhibitors that are able to target all KRAS mutations by inhibiting the SOS1 (Son of Sevenless 1) molecule, an upstream effector of KRAS. Particularly, the authors have shown that a combination of Omomyc and the KRAS G12C inhibitors sotorasib or adagrasib synergistically decreases the viability of NSCLC cell lines HCC44, H1792, H23 and H358; and also the viability of colorectal cancer (CRC) cell line SW1463 (Figures 1 and 2). The combination of Omomyc with the KRAS G12D inhibitor MRTX1133 is also synergic in NSCLC cell lines KLA-p53 and KLA-Stk11, and in CRC cell line Lim1215 (Figure 3).

The authors have also discovered that this synergic effect is maintained when KRAS inhibitors that target the KRAS pathway regardless of the specific KRAS mutation are used. Figures 4 and 5 show that a combination of Omomyc and the pan-KRAS (SOS1) inhibitors BAY-293 or BI-3406 synergistically decreases the viability of NSCLC cell lines H358, HCC44 and KLA-p53, which are KRASG12C or KRASG12D mutants; and also the viability of colorectal cancer (CRC) cell line SW1463.

This synergistic effect is maintained regardless the dose (Figures 1-5) resulting in a beneficial effect, in particular, an increase in the therapeutic effect of the composition of the invention relative to each of its components so that it can reach the same result with lower doses of each of the components, thereby reducing the side effects on the subject receiving the composition of the invention.

Furthermore, the inventors have surprisingly found that Omomyc is able to revert KRAS-resistance, thus enhancing sensitivity to KRAS inhibitors and overcoming KRAS inhibitors resistance, which is one of the main drawbacks of the treatment with KRAS inhibitors. The combination of the invention broadens the population who may be responsive to a therapy based on KRAS inhibitors.

Therefore, a combination of Omomyc with a KRAS inhibitor can be an effective therapy for the treatment of both KRAS-mutated and wild-type tumors, and for treating tumors resistant to KRAS inhibitors.

Thus, in a first aspect, the invention relates to a combination comprising:
i) a first component selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b);
   and
ii) a second component that is a KRAS inhibitor.

According to the invention, the expression "combination" stands for the various combinations of compounds (i) and (ii), for example in a composition formulated as a single formulation, in a combined mixture composed from separate formulations of each of the components, such as a "tank-mix" which may be combined for joint use as a combined preparation, and in a combined use of the single active ingredients when applied in a sequential manner, i.e., one after the other with a reasonably short period, such as a few hours or days or in simultaneous administration. In the present invention, compound (i) refers to a therapeutically effective amount of a polypeptide comprising the sequence SEQ ID NO: 1 or a functionally equivalent variant thereof, or refers to a conjugate comprising a polypeptide comprising the sequence SEQ ID NO: 1 or a functionally equivalent variant thereof and a chemical moiety that facilitates cellular uptake of the polypeptide or of the functionally equivalent variant thereof, or refers to a polynucleotide encoding the polypeptide or the conjugate, or refers to a vector comprising the polynucleotide, or refers to a cell capable of secreting into the medium the polypeptide or the conjugate. In the present invention, compound (ii) refers to a therapeutically effective amount of a KRAS inhibitor. Preferably, the order of applying the compounds (i) and (ii) is not essential for working the present invention.

The combination may be a kit-of-parts wherein each of the components is individually formulated and packaged.

A combination of compounds (i) and (ii) can be formulated for its simultaneous, separate or sequential administration. Particularly, if the administration is not simultaneous, the compounds are administered in a close time proximity to each other. Furthermore, compounds are administered in the same or different dosage form or by the same or different administration route, e.g. one compound can be administered orally and the other compound can be administered intravenously. Preferably, compound (i) is administered intravenously and compound (ii) is administered orally. In another embodiment, compound (i) is administered intranasally and compound (ii) is administered orally. In another embodiment, compounds (i) and (ii) are administered intravenously.

The combination of the two compounds (i) and (ii) can be administered:
- as a combination that is being part of the same medicament formulation, the two compounds being then administered always simultaneously.
- as a combination of two units, each with one of the substances giving rise to the possibility of simultaneous, sequential or separate administration.

In a particular embodiment, compound (i) of the combination of the invention is independently administered from compound (ii), i.e. in two units, but at the same time.

In another particular embodiment, compound (i) of the combination of the invention is administered first, and then compound (ii), i.e. the compound (ii) is separately or sequentially administered.

In yet another particular embodiment, compound (ii) of the combination of the invention is administered first, and then compound (i), i.e. the compound (i) is administered separately or sequentially, as defined.

If administered separately, compounds (i) and (ii) of the combination of the invention can be administered within a period of time from one another, for example, within 1, 2, 3, 4, 5, 6, 7, 8, 9, 10, 11, 12, 13, 14, 15, 16, 17, 18, 19, 20, 21, 22, 23, or 24 hours from one another. In another embodiment, compounds (i) and (ii) of the combination of the invention can be administered within 1, 2, 3, 4, 5, 6, 7, 8, 9, 10, 11, 12, 13, 14, 15, 16, 17, 18, 19, 20, 21, 22, 23, or 24 days from one another, preferably within 1 days from one another, more preferably within 10 days from one another. In a preferred embodiment, compound (ii) is administered after 10 days of the first administration of compound (i). In an embodiment the administration of the first compound is discontinued before starting the administration of the second compound.

In another aspect, the invention relates to a combination or pharmaceutical composition comprising a synergistically effective amount of a first component according to the first aspect of the invention and a KRAS inhibitor.

### Compound (i) of the combination of the invention

In a preferred embodiment, compound (i) of the invention is a polypeptide comprising the sequence SEQ ID NO: 1 or a functionally equivalent variant thereof; more preferably a polypeptide comprising the sequence SEQ ID NO: 1.

The terms "polypeptide" and "peptide" are used interchangeably herein to refer to polymers of amino acids of any length. The polypeptide of the invention can comprise modified amino acids, and it can be interrupted by non-amino acids. In a preferred embodiment the polypeptide is exclusively formed by amino acids. Preferably the polypeptide that forms item (i) of the combination has a length between 80 and 500 amino acids, more preferably between 80 and 300 amino acids, more preferably between 80 and 250 amino acids, more preferably between 80 and 150, even more preferably between 80 and 130 amino acids, preferably between 90 and 130 amino acids, preferably no more than 125 amino acids, more preferably no more than 100 amino acids. In a preferred embodiment, the polypeptide has a length between 90 and 98 amino acids, preferably between 90 and 95 amino acids, more preferably 91 amino acids.

The term "amino acid" refers to naturally occurring and synthetic amino acids, as well as amino acid analogs and amino acid mimetics that function in a manner similar to the naturally occurring amino acids. Furthermore, the term "amino acid" includes both D- and L-amino acids (stereoisomers). Preferably, the amino acids are L-amino acids.

The term "natural amino acids" or "naturally occurring amino acids" comprises the 20 naturally occurring amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, nor-valine, nor-leucine and ornithine.

As used herein, the term "non-natural amino acid" or "synthetic amino acid" refers to a carboxylic acid, or a derivative thereof, substituted at position "a" with an amine group and being structurally related to a natural amino acid. Illustrative non- limiting examples of modified or uncommon amino acids include 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxy lysine, alio hydroxy lysine, 3-hydroxyproline, 4-hydroxyproline, isodesmosine, alloisoleucine, N-methylglycine, N-methylisoleucine, 6-N-methyl-lysine, N-methylvaline, norvaline, norleucine, ornithine, etc.

The polypeptide of the present invention may also comprise non-amino acid moieties, such as for example, hydrophobic moieties (various linear, branched, cyclic, polycyclic or heterocyclic hydrocarbons and hydrocarbon derivatives) attached to the peptides; various protecting groups which are attached to the compound's terminals to decrease degradation. Suitable protecting functional groups are described in Green and Wuts, "Protecting Groups in Organic Synthesis", John Wiley and Sons, Chapters 5 and 7, 1991.

Chemical (non-amino acid) groups present in the polypeptide may be included in order to improve various physiological properties such as decreased degradation or clearance; decreased repulsion by various cellular pumps, improve various modes of administration, increased specificity, increased affinity, increased stability, bioavailability, solubility, decreased toxicity and the like.

"Mimetic" include molecules which mimic the chemical structure of a peptidic structure and retain the functional properties of the peptidic structure. Approaches to designing peptide analogs, derivatives and mimetics are known in the art.

In an embodiment the polypeptide of the invention is a polypeptide consisting of sequence SEQ ID NO: 1 or a polypeptide consisting of a functionally equivalent variant of SEQ ID NO: 1, preferably is a polypeptide consisting of the sequence SEQ ID NO: 1. The SEQ ID NO: 1 corresponds to

TEENVKRRTHNVLERQRRNELKRSFFALRDQIPELENNEKAPKVVILKKATAYILSVQA
ETQKLISEIDLLRKQNEQLKHKLEQLRNSCA (SEQ ID NO: 1)

The polypeptide of sequence SEQ ID NO: 1 corresponds to the Omomyc protein sequence. The term "Omomyc", as used herein, refers to a polypeptide which consists of a mutated version of the bHLHZip domain of the Myc carrying the E61T, E68I, R74Q and R75N mutations (wherein the numbering of the mutated positions is given with respect to the sequence of Myc region corresponding to amino acids 365-454 of the polypeptide as defined under accession number NP_002458 in the NCBI database, release of March 15, 2015). The sequence of c-Myc provided in the NCBI database under the accession number NP_002458 is shown below (SEQ ID NO: 2), wherein the region from which Omomyc derives is shown underlined:

1 MDFFRVVENQ QPPATMPLNV SFTNRNYDLD YDSVQPYFYC DEEENFYQQQ QQSELQPPAP

61 SEDIWKKFEL LPTPPLSPSR RSGLCSPSYV AVTPFSLRGD NDGGGGSFST ADQLEMVTEL

121 LGGDMVNQSF ICDPDDETFI KNIIIQDCMW SGFSAAAKLV SEKLASYQAA RKDSGSPNPA

181 RGHSVCSTSS LYLQDLSAAA SECIDPSVVF PYPLNDSSSP KSCASQDSSA FSPSSDSLLS

241 STESSPQGSP EPLVLHEETP PTTSSDSEEE QEDEEEIDVV SVEKRQAPGK RSESGSPSAG

301 GHSKPPHSPL VLKRCHVSTH QHNYAAPPST RKDYPAAKRV KLDSVRVLRQ ISNNRKCTSP

361 RSSDTEENVK RRTHNVLERQ RRNELKRSFF ALRDQIPELE NNEKAPKVVI LKKATAYILS

421 VQAEEQKLIS EEDLLRKRRE QLKHKLEQLR NSCA (SEQ ID NO: 2)

Omomyc also contains the M2 domain of c-Myc, having the sequence RQRRNELKRSF (SEQ ID NO: 3) (see Dang and Lee, Mol.Cell. Biol., 1988, 8:4048-4054) (double underlined above), and which corresponds to a nuclear localization signal.

Omomyc is characterized in that it shows increased dimerization capacity with all three oncogenic Myc proteins (c-Myc, N-Myc and L-Myc). Omomyc can derive from the bHLHZip domain of any Myc protein known in the art, provided that the mutations which result in the tumor suppressor effect are preserved. Thus, the Omomyc that can be used in the present invention may derive from any mammal species, including but not being limited to domestic and farm animals (cows, horses, pigs, sheep, goats, dog, cats or rodents), primates and humans. Preferably, the Omomyc protein is derived from human Myc protein (accession number NP_002458, release of March 12, 2019).

The term "Myc", as used, herein, refers to a family of transcription factors which includes c-Myc, N-Myc and L-Myc. Myc protein activates expression of many genes through binding on consensus sequence CACGTG (Enhancer Box sequences or E-boxes and recruiting histone acetyl-transferases or HATs). However, Myc can also act as a transcriptional repressor. By binding the Miz-1 transcription factor and displacing p300 co-activator, it inhibits expression of Miz-1 target genes. Myc also has a direct role in the control of DNA replication.

The Myc b-HLH-LZ or Myc basic region helix-loop-helix leucine zipper domain refers to a region which determines Myc dimerization with Max protein and binding to Myc-target genes. This region corresponds to amino acids 365-454 of human Myc and is characterized by two alpha helices connected by a loop (Nair, S. K., & Burley, S. K., 2003, Cell, 112: 193-205).

In a preferred embodiment, the polypeptide of the invention is a polypeptide that comprises, consists of or consists essentially of the SEQ ID NO: 4 shown below.

MTEENVKRRTHNVLERQRRNELKRSFFALRDQIPELENNEKAPKVVILKKATAYILSVQ
AETQKLISEIDLLRKQNEQLKHKLEQLRNSCA (SEQ ID NO: 4)

In this context, "consisting essentially of" means that the specified molecule would not contain any additional sequences that would alter the activity of SEQ ID NO: 4.

Preferably, the polypeptide consists of SEQ ID NO: 4.

The term "functionally equivalent variant", refers to any polypeptide which results from the insertion or addition of one or more amino acids and/or from the deletion of one or more amino acids and/or from the conservative substitution of one or more amino acids with respect to the polypeptide of SEQ ID NO: 1 and/or which results from the chemical modification of the polypeptide of SEQ ID NO: 1 and which substantially preserves the tumor suppressor activity of the SEQ ID NO: 1. Preferably, the functionally equivalent variant refers to any polypeptide which results from the insertion or addition of one or more amino acids and/or from the deletion of one or more amino acids and/or from the conservative substitution of one or more amino acids with respect to the polypeptide of SEQ ID NO: 1 and which substantially preserves the tumor suppressor activity of SEQ ID NO: 1; more preferably results from the insertion or addition of one or more amino acids with respect to the polypeptide of SEQ ID NO: 1.

The skilled person will understand that the preservation of the tumor suppressor activity requires that the variant can dimerize with Myc and/or its obligate partner p21/p22Max and inhibit Myc activity, that it is capable of translocating across the cell membrane and that it is capable of translocating across the nuclear envelope. In some embodiments, the functionally equivalent variant of the polypeptide of the invention homodimerizes less than Omomyc, or is not forced into homodimers by the formation of disulphide bridge. In particular the disulphide bridge formation in the homodimer form of certain embodiments of the polypeptide of the invention is less than in the polypeptide Omomyc.

"Less homodimerization", as used herein, relates to the lower ability of forming obligate homodimers of the polypeptide of the invention even in reducing conditions. In a preferred embodiment, the ability is at least 5%, at least 10%, at least 15%, at least 20%, at least 25%, at least 30%, at least 35%, at least 40%, at least 45 %, at least 50%, at least 55%, at least 60%, at least 65%, at least 70%, at least 75%, at least 80%, at least 85%, at least 90%, at least 95% less than the ability of forming homodimers of Omomyc.

Reducing conditions, as used herein, relates to the presence of a reducing agent, a compound that donates an electron to another chemical species in a redox chemical reaction. Illustrative, non-limitative examples of reducing agents are DTT (dithiothreitol), b-mercaptoethanol or TCEP (tris(2-carboxyethyl)phosphine). It is possible that the amount of homodimers is the same *in vitro,* and that the difference between the functionally equivalent variant and Omomyc is present only in cells in presence of heterodimerization partners where the absence of the disulfide enables a potentially higher formation of heterodimers.

Several assays may be used to determine the homodimerization of a peptide, by way of illustrative non-limitative example by thermal denaturation monitored by circular dichroism, so dimerization may be detected through folding and thermal stability quantification.

Suitable functionally equivalent variants include polypeptides consisting essentially of the polypeptide of SEQ ID NO: 1. In this context, "consisting essentially of' means that the specified molecule would not contain any additional sequences that would alter the activity of the SEQ ID NO: 1.

In a preferred embodiment, the functionally equivalent variant of SEQ ID NO: 1 is a polypeptide which results from the insertion or addition of one or more amino acids with respect to the polypeptide of SEQ ID NO: 1. In an embodiment, the functionally equivalent variant results from the insertion of less than 10 amino acids, more preferably less than 5 amino acids, more preferably results from the insertion of one amino acid. In a preferred embodiment, results from the insertion of one amino acid that is methionine.

In another embodiment, the functionally equivalent variant of SEQ ID NO: 1 is a polypeptide which results from the deletion of one or more amino acids with respect to the polypeptide of SEQ ID NO: 1. In an embodiment, the functionally equivalent variant results from the deletion of less than 10 amino acids, more preferably less than 5 amino acids, more preferably results from the deletion of one amino acid.

Suitable functional variants of the targeting peptide are those showing a degree of identity with respect to the peptide of SEQ ID NO:1 of about greater than 25% amino acid sequence identity, such as 25%, 30%, 40%, 50%, 60%, 65%, 70%, 75%, 80%, 85%, 90%, 91%, 92%, 93%, 94%, 95%, 96%, 97%, 98% or 99%. The degree of identity between two polypeptides is determined using computer algorithms and methods that are widely known for the persons skilled in the art. The identity between two amino acid sequences is preferably determined by using the BLASTP algorithm as described previously (BLAST Manual, Altschul, S., et al., NCBI NLM NIH Bethesda, Md. 20894, Altschul, S., et al., J. Mol. Biol. 1990;215: 403-410). In a preferred embodiment, the sequence identity is determined throughout the whole length of the polypeptide of SEQ ID NO: 1 or throughout the whole length of the variant or of both.

The functionally equivalent variants of the polypeptide of the invention may also include post-translational modifications, such as glycosylation, acetylation, isoprenylation, myristoylation, proteolytic processing, etc.

In another embodiment, suitable functional variants of the targeting peptide are those wherein one or more positions within the polypeptide of the invention contain an amino acid which is a conservative substitution of the amino acid present in the protein mentioned above. "Conservative amino acid substitutions" result from replacing one amino acid with another having similar structural and/or chemical properties. For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example, by Dordo et al., (J. Mol. Biol, 1999, 217;721-739) and Taylor et al., (J. Theor. Biol., 1986, 119:205-218).

It will be understood that in a preferred embodiment the functionally equivalent variants of Omomyc contain mutations at positions corresponding to the mutations E61T, E68I, R74Q and R75N found in Omomyc derived from human c-Myc. The position wherein said mutations have to occur in the functionally equivalent variant can be determined by a multiple sequence alignment of different Myc sequences and identifed by the alignment of those positions corresponding to positions 61, 68, 74 and 75 within the sequence of Omomyc derived from human c-Myc. In an embodiment, the functionally equivalent variants of Omomyc contain mutations at positions corresponding to the mutations E61T, E68I, R74Q and R75N found in Omomyc derived from human c-Myc.

In another embodiment, the functionally equivalent variants of Omomyc contain mutations at positions corresponding to E61, E68, R74 and R75 within the sequence of Omomyc wherein E61 has been mutated to E61A or E61S; E68 has been mutated to E68L, E68M or E68V; R74 has been mutated to R74N; and R75 has been mutated to R75Q.

A multiple sequence alignment is an extension of pairwise alignment to incorporate more than two sequences at a time. Multiple alignment methods align all of the sequences in a given query set. A preferred multiple sequence alignment program (and its algorithm) is ClustalW, Clusal2W or ClustalW XXL (see Thompson et al. (1994) Nucleic Acids Res 22:4673-4680). Once the sequences of c-Myc from different organisms and of the variant are compared (aligned) as described herein, the skilled artisan can readily identify the positions within each of the sequence corresponding to positions E61T, E68I, R74Q and R75N found in Omomyc and introduce within the Omomyc variant mutations corresponding to the E61T, E68I, R74Q and R75N mutations found in Omomyc derived from human c-Myc.

Suitable assays for determining whether a polypeptide can be considered as a functionally equivalent variant of Omomyc include, without limitation:
- Assays which measure the capacity of the polypeptide to form dimeric complexes with Max and Myc, such as the assays based on the expression of a reporter gene as described in Soucek et al. (Oncogene, 1998, 17: 2463 - 2472) as well as PLA (protein Ligation assay) or co-immunoprecipitation.
- Assays which measure the capacity of the polypeptide to bind to the Myc/Max recognition site within DNA (the CACGTG site), such as the electrophoretic mobility shift assay (EMSA) described in Soucek et al. (supra.)
- Assays which measure the capacity to repress Myc-induced transactivation, such as the assay based on the expression of a reporter gene under the control of the DNA binding sites specific for Myc/Max as described by Soucek et al. (supra.).
- Assays based on the capacity of the polypeptide to inhibit growth of cells expressing the myc oncogene, as described by Soucek et al. (supra.).
- Assays which measure the ability of the polypeptide to enhance myc-induced apoptosis, such as the assays described by Soucek et al. (Oncogene, 1998: 17, 2463 - 2472). Moreover, any assay commonly known in the art for assessing apoptosis in a cell can be used, such as the Hoechst staining, Propidium Iodide (PI) or Annexin V staining, trypan blue, DNA laddering/fragmentation and TUNEL.

In a preferred embodiment, a polypeptide is considered a functionally equivalent variant of Omomyc if it shows an activity in one or more of the above assays which is at least 10%, 20%, 30%, 40%, 50%, 60%, 70%, 80%, 90% or 100% of the native Omomyc.

In a particular embodiment, the functionally equivalent variant of the polypeptide of SEQ ID NO: 1 comprises the polypeptide of SEQ ID NO: 1, wherein the residue X at position 89 of SEQ ID NO: 1 is not a cysteine. Preferably, the residue X at position 89 of SEQ ID NO: 1 is an aliphatic amino acid, or a sulfured amino acid, or a dicarboxylic amino acid or their amides, or an amino acid having two basic groups, or an aromatic amino acid, or a cyclic amino acid, or a hydroxylated amino acid. More preferably is an amino acid selected from serine, threonine and alanine, preferably selected from serine and alanine.

Suitable functionally equivalent variants of SEQ ID NO: 1 having a residue X at position 89 of SEQ ID NO: 1 which is not a cysteine are disclosed in the following table.

| **SEQ ID NO** | **SEQUENCE** |
|---|---|
| SEQ ID NO: 5 | TEENVKRRTHNVLERQRRNELKRSFFALRDQIPELENNEKAPKVVILKKATAYILSV QAETQKLISEIDLLRKQNEQLKHKLEQLRNSXA (wherein X is any aa different from Cys) |
| SEQ ID NO: 6 | MTEENVKRRTHNVLERQRRNELKRSFFALRDQIPELENNEKAPKVVILKKATAYILS VQAETQKLISEIDLLRKQNEQLKHKLEQLRNSXA (wherein X is any aa different from Cys) |
| SEQ ID NO: 7 | TEENVKRRTHNVLERQRRNELKRSFFALRDQIPELENNEKAPKVVILKKATAYILSV QAETQKLISEIDLLRKQNEQLKHKLEQLRNSSA |
| SEQ ID NO: 8 | MTEENVKRRTHNVLERQRRNELKRSFFALRDQIPELENNEKAPKVVILKKATAYILS VQAETQKLISEIDLLRKQNEQLKHKLEQLRNSSA |
| SEQ ID NO: 9 | TEENVKRRTHNVLERQRRNELKRSFFALRDQIPELENNEKAPKVVILKKATAYILSV QAETQKLISEIDLLRKQNEQLKHKLEQLRNSAA |
| SEQ ID NO: 10 | MTEENVKRRTHNVLERQRRNELKRSFFALRDQIPELENNEKAPKVVILKKATAYILS VQAETQKLISEIDLLRKQNEQLKHKLEQLRNSAA |

Thus, in a preferred embodiment, the functionally equivalent variant of the polypeptide of SEQ ID NO: 1 is selected from the group consisting of SEQ ID NO: 4, SEQ ID NO: 5, SEQ ID NO: 6, SEQ ID NO: 7, SEQ ID NO: 8, SEQ ID NO: 9 and SEQ ID NO: 10. Preferably, the functionally equivalent variant is SEQ ID NO: 4.

Additionally, functionally equivalent variants of Omomyc are also capable of transducing cells after the variant is contacted with said cell. It will be understood that functionally equivalent variants of Omomyc contain the protein transducing domain found in native Omomyc or another functional protein transducing domain.

In a preferred embodiment, a polypeptide is considered as a functionally equivalent variant of SEQ ID NO: 1 if it is capable of transducing a target cell at least 10%, 20%, 30%, 40%, 50%, 60%, 70%, 80%, 90% or 100% as efficiently as SEQ ID NO: 1.

Additionally, functionally equivalent variants of SEQ ID NO: 1 are also capable of translocating to the nucleus of the target tumor cell.

In a preferred embodiment, a polypeptide is considered as a functionally equivalent variant of SEQ ID NO: 1 if it is capable of translocating to the nucleus of the target tumor cells at least 10%, 20%, 30%, 40%, 50%, 60%, 70%, 80%, 90% or 100% as efficiently as the SEQ ID NO: 1.

Suitable assays for determining whether a polypeptide is a functionally equivalent variant of SEQ ID NO: 1 in terms of its ability to translocate across the cellular membrane and to the nucleus include double labelling of a cell with a reagent specific for the polypeptide and with a dye which specifically labels the nucleus of the cell (such as DAPI or Hoechst dye). The detection of the polypeptide of the invention can be performed by confocal microscopy or by fluorescence microscopy.

In another preferred embodiment, compound (i) of the invention is a conjugate comprising a polypeptide comprising the sequence SEQ ID NO: 1 or a functionally equivalent variant thereof and a chemical moiety that facilitates cellular uptake of the polypeptide or of the functionally equivalent variant thereof.

The term "conjugate", as used herein, refers to two or more compounds which are covalently linked together so that the function of each compound is retained in the conjugate.

The term "chemical moiety" refers to any chemical compound containing at least one carbon atom. Examples of chemical moieties include, but are not limited to, any peptide chain enriched in hydrophobic amino acids and hydrophobic chemical moieties.

In preferred embodiments, the conjugate according to the invention comprises at least 1, at least 2, at least 3, at least 4, at least 5, at least 6, at least 7, at least 8, at least 9, at least 10 or more chemical moieties that facilitate cellular uptake of the polypeptide or of the functionally equivalent variant of said polypeptide.

In one embodiment, the chemical moiety that facilitates cellular uptake of the polypeptide is a lipid or a fatty acid.

A fatty acid generally is a molecule comprising a carbon chain with an acidic moiety (e.g., carboxylic acid) at an end of the chain. The carbon chain of a fatty acid may be of any length, however, it is preferred that the length of the carbon chain be of at least 2, 3, 4, 5, 6, 7, 8, 9, 10, 11, 12, 13, 14, 15, 16, 17, 18, 19, 20 or more carbon atoms, and any range derivable therein. In certain embodiments, the length of the carbon chain is from 4 to 18 carbon atoms in the chain portion of the fatty acid. In certain embodiments the fatty acid carbon chain may comprise an odd number of carbon atoms, however, an even number of carbon atoms in the chain may be preferred in certain embodiments. A fatty acid comprising only single bonds in its carbon chain is called saturated, while a fatty acid comprising at least one double bond in its chain is called unsaturated. The fatty acid may be branched, though in preferable embodiments of the present invention, it is unbranched. Specific fatty acids include, but are not limited to, linoleic acid, oleic acid, palmitic acid, linolenic acid, stearic acid, lauric acid, myristic acid, arachidic acid, palmitoleic acid, and arachidonic acid.

In a preferred embodiment, the chemical moiety that facilitates the cellular uptake of the polypeptide comprising the sequence SEQ ID NO: 1 or a functionally equivalent variant thereof, is a cell penetrating peptide sequence, in which case, the conjugate would comprise a fusion protein comprising the polypeptide comprising SEQ ID NO: 1 or the functionally equivalent variant thereof and the cell penetrating peptide sequence.

The term "fusion protein" relates to proteins generated by gene technology which consist of two or more functional domains derived from different proteins. A fusion protein may be obtained by conventional means, e.g., by means of gene expression of the nucleotide sequence encoding for said fusion protein in a suitable cell. It will be understood that the cell penetrating peptide refers to a cell penetrating peptide which is different from the cell penetrating peptide which forms part of the polypeptide comprising SEQ ID NO: 1 or of the functionally equivalent variant of SEQ ID NO: 1.

The term "cell penetrating peptide sequence" is used in the present specification interchangeably with "CPP", "protein transducing domain" or "PTD". It refers to a peptide chain of variable length that directs the transport of a protein inside a cell. The delivering process into cell commonly occurs by endocytosis but the peptide can also be internalized into cell by means of direct membrane translocation. CPPs typically have an amino acid composition that either contains a high relative abundance of positively charged amino acids such as lysine or arginine or has sequences that contain an alternating pattern of polar/charged amino acid and non-polar, hydrophobic amino acids.

Examples of CPPs which can be used in the present invention include, without limitation, the CPP found in Drosophila antennapedia protein (RQIKIWFQNRRMKWKK. SEQ ID NO:13), the CPP found in the herpesvirus simplex 1 (HSV-1) VP22 DNA-binding protein (DAATATRGRSAASRPTERPRAPARSASRPRRPVE, SEQ ID NO:14) , the CPP of Bac-7 (RRIRPRPPRLPRPRPRPLPFPRPG; SEQ ID NO: 15), the CPPs of the HIV-1 TAT protein consisting of amino acids 49-57 (RKKRRQRRR, SEQ ID NO: 16), amino acids 48-60 (GRKKRRQRRRTPQ, SEQ ID NO: 17), amino acids 47-57 (YGRKKRRQRRR; SEQ ID NO: 18); the CPP of S413-PV peptide (ALWKTLLKKVLKAPKKKRKV; SEQ ID NO: 19), the CPP of penetratin (RQIKWFQNRRMKWKK; SEQ ID NO: 20), the CPP of SynB1 (RGGRLSYSRRRFSTSTGR; SEQ ID NO: 21), the CPP of SynB3 (RRLSYSRRRF; SEQ ID NO: 22), the CPP of PTD-4 (PIRRRKKLRRLK; SEQ ID NO: 23), the CPP of PTD-5 (RRQRRTSKLMKR; SEQ ID NO: 24), the CPP of the FHV Coat-(35-49) (RRRRNRTRRNRRRVR; SEQ ID NO: 25), the CPP of BMV Gag-(7-25) (KMTRAQRRAAARRNRWTAR; SEQ ID NO: 26), the CPP of HTLV-II Rex-(4-16) (TRRQRTRRARRNR; SEQ ID NO: 27), the CPP of D-Tat (GRKKRRQRRRPPQ; SEQ ID NO:28), the CPP R9-Tat (GRRRRRRRRRPPQ; SEQ ID NO: 29), the CPP of MAP (KLALKLALKLALALKLA; SEQ ID NO: 30), the CPP of SBP (MGLGLHLLVLAAALQGAWSQPKKKRKV; SEQ ID NO: 31), the CPP of FBP (GALFLGWLGAAGSTMGAWSQPKKKRKV; SEQ ID NO: 32), the CPP of MPG (ac-GALFLGFLGAAGSTMGAWSQPKKKRKV-cya; SEQ ID NO: 33), the CPP of MPG(ENLS) (ac-GALFLGFLGAAGSTMGAWSQPKSKRKV-cya; SEQ ID NO: 34), the CPP of Pep-1 (ac-KETWWETWWTEWSQPKKKRKV-cya; SEQ ID NO: 35), the CPP of Pep-2 (ac-KETWFETWFTEWSQPKKKRKV-cya; SEQ ID NO: 36), a polyarginine sequence having the structure RN (wherein N is between 4 and 17), the GRKKRRQRRR sequence (SEQ ID NO: 37), the RRRRRRLR sequence (SEQ ID NO: 38), the RRQRRTS KLMKR sequence (SEQ ID NO: 39); Transportan GWTLNSAGYLLGKINLKALAALAKKIL (SEQ ID NO: 40); KALAWEAKLAKALAKALAKHLAKALAKALKCEA (SEQ ID NO: 41); RQIKIWFQNRRMKWKK (SEQ ID NO: 42), the YGRKKRRQRRR sequence (SEQ ID NO: 43); the RKKRRQRR sequence (SEQ ID NO: 44); the YARAAARQARA sequence (SEQ ID NO: 45); the THRLPRRRRRR sequence (SEQ ID NO: 46); the GGRRARRRRRR sequence (SEQ ID NO: 47).

In a preferred embodiment, said cell-penetrating peptide is not the endogenous contained in SEQ ID NO: 1.

In a preferred embodiment, the CPP is the CPP of the HIV-1 TAT protein consisting of amino acids 49-57 (RKKRRQRRR, SEQ ID NO: 16). In another preferred embodiment the CPP is the GRKKRRQRRR sequence (SEQ ID NO: 37) or RRRRRRLR (SEQ ID NO: 38). In another embodiment, the CPP is the GRKKRRQRRR sequence (SEQ ID NO: 37) or RRRRRRRR (SEQ ID NO: 65).

In some embodiments, a CPP is as a CPP as described in WO2019/018898, the content of which is incorporated herein by reference in its entirety.

In one embodiment, the cell-penetrating peptide sequence is fused at the N-terminus of the polypeptide of the invention or of the functionally equivalent variant of said polypeptide. In another embodiment, the cell-penetrating peptide is fused at the C-terminus of the polypeptide of the invention or of the functionally equivalent variant of said polypeptide.

In preferred embodiments, the conjugates or fusion proteins of the combination according to the invention comprise, in addition to the own cell penetrating peptide found in the polypeptide of SEQ ID NO: 1 or of the functionally equivalent variant of said polypeptide, at least 1, at least 2, at least 3, at least 4, at least 5, at least 6, at least 7, at least 8, at least 9, at least 10 or more additional cell penetrating peptides.

Suitable fusion proteins of the invention include the polypeptides Omomyc*TAT and Omomyc*LZArg as defined below:

| Name | SEQ ID NO: | Sequence |
|---|---|---|
| Omomyc^{∗}TAT | 11 | MTEENVKRRTHNVLERQRRNELKRSFFALRDQIPELENNEKAPKVVI LKKATAYILSVQAETQKLISEIDLLRKQNEQLKHKLEQLRNSCAGRK KRRQRRR |
| Omomyc^{∗}LZArg | 12 | MTEENVKRRTHNVLERQRRNELKRSFFALRDQIPELENNEKAPKVVI LKKATAYILSVQAETQKLISEIDLLRKQNEQLKHKLEQLRNSCARRR RRRLR |

Thus, in a preferred embodiment the fusion protein is the polypeptide selected from SEQ ID NO: 11 and 12.

Suitable assays for determining whether a conjugate preserves the cell membrane translocation capacity of Omomyc include, without limitation, assays which measure the capacity of the conjugate to transduce cells in culture. This assay is based on contacting the conjugate with culture cells and detecting the presence of the conjugate in an intracellular location.

In another preferred embodiment, the conjugate of the combination of the invention additionally comprises a further nuclear localization signal.

The term "nuclear localization signal" (NLS), as used herein, refers to an amino acid sequence of about 4-20 amino acid residues in length, which serves to direct a protein to the nucleus. Typically, the nuclear localization sequence is rich in basic amino acids and exemplary sequences are well known in the art (Gorlich D. (1998) EMBO 5.17:2721-7). In some embodiments, the NLS is selected from the group consisting of the SV40 large T Antigen NLS (PKKKRKV, SEQ ID NO: 48); the Nucleoplasmin NLS (KRPAATKKAGQAKKKK, SEQ ID NO: 49); the CBP80 NLS (RRRHSDENDGGQPHKRRK, SEQ ID NO: 50); the HIV-I Rev protein NLS (RQARRNRRRWE, SEQ ID NO: 51); the HTLV-I Rex (MPKTRRRPRRSQRKRPPT, SEQ ID NO: 52); the hnRNP A NLS (NQSSNFGPMKGGNFGGRSSGPYGGGGQYFKPRNQGGY, SEQ ID NO: 53); the rpL23a NLS (VHSHKKKKIRTSPTFTTPKTLRLRRQPKYPRKSAPRRNKLDHY, SEQ ID NO: 54). In one embodiment of the invention, the nuclear localization signal comprises the motif K (K/ R) X (K/ R).

In an even more preferred embodiment, the nuclear localization signal is selected from the group consisting of PKKKRKV (SEQ ID NO: 48), PAAKRVKLD (SEQ ID NO: 56) and KRPAATKKAGQ AKKKK (SEQ ID NO: 49).

In another preferred embodiment, the NLS may be N-terminal or C-terminal to the conjugate or the fusion protein comprising the polypeptide of SEQ ID NO: 1 or a functionally equivalent variant thereof.

The skilled person will understand that it may be desirable that the conjugate of the invention further comprises one or more flexible peptides that connect the polypeptide comprising SEQ ID NO: 1 or the functionally equivalent variant thereof, the cell penetrating peptide sequence and/or the NLS. Thus, in a particular embodiment the polypeptide comprising SEQ ID NO: 1 or a functionally equivalent variant thereof is directly connected to the cell penetrating peptide sequence. In another particular embodiment, the polypeptide comprising SEQ ID NO: 1 or a functionally equivalent variant thereof is connected to the cell penetrating peptide sequence through a flexible peptide. In an embodiment the polypeptide comprising SEQ ID NO: 1 or the functionally variant thereof is directly connected to the NLS. In another embodiment the polypeptide comprising SEQ ID NO: 1 or a functionally equivalent variant thereof is connected to the NLS through a flexible peptide.

In a particular embodiment the polypeptide of the conjugate according to the invention is directly connected to the cell penetrating peptide sequence and to the NLS.

In one embodiment, the NLS is one of the NLS which appears endogenously in the Myc sequence, such as the M1 peptide (PAAKRVKLD, SEQ ID NO: 56) or the M2 peptide (RQRRNELKRSF, SEQ ID NO: 57).

In another embodiment the additional NLS refers to an NLS which is different to the endogenous NLS found in a polypeptide comprising SEQ ID NO: 1 or in the functionally equivalent variant of SEQ ID NO: 1.

In preferred embodiments, the conjugates or fusion proteins according to the invention comprise, in addition to the endogenous NLS found in the polypeptide of the invention or in the functionally equivalent variant thereof, at least 1, at least 2, at least 3, at least 4, at least 5, at least 6, at least 7, at least 8, at least 9, at least 10 NLS.

In another particular embodiment, the polypeptide of the conjugate fore use according to the invention is connected to the cell penetrating peptide sequence through a first flexible peptide linker and to the NLS through a second flexible peptide linker.

As used herein, the term "flexible peptide", "spacer peptide" or "linker peptide" refers to a peptide that covalently binds two proteins or moieties but which is not part of either polypeptide, allowing movement of one with respect to the other, without causing a substantial detrimental effect on the function of either the protein or the moiety. Thus, the flexible linker does not affect the tumour tracing activity of the polypeptide sequence, the cell penetrating activity of the cell penetrating peptide or the nuclear localization capacity of the NLS.

The flexible peptide comprises at least one amino acid, at least two amino acids, at least three amino acids, at least four amino acids, at least five amino acids, at least six amino acids, at least seven amino acids, at least eight amino acids, at least nine amino acids, at least 10 amino acids, at least 12 amino acids, at least 14 amino acids, at least 16 amino acids, at least 18 amino acids, at least 20 amino acids, at least 25 amino acids, at least 30 amino acids, at least 35 amino acids, at least 40 amino acids, at least 45 amino acids, at least 50 amino acids, at least 60 amino acids, at least 70 amino acids, at least 80 amino acids, at least 90 amino acids, or about 100 amino acids. In some embodiments the flexible peptide will permit the movement of one protein with respect to the other in order to increase solubility of the protein and/or to improve its activity. Suitable linker regions include a poly-glycine region, the GPRRRR sequence (SEQ ID NO: 58) of combinations of glycine, proline and alanine residues.

In a particular embodiment, the conjugates according to the invention comprise a tag bound to the conjugate or to the C-terminal or N-terminal domain of said polypeptide or fusion protein or variant thereof. Said tag is generally a peptide or amino acid sequence which can be used in the isolation or purification of said fusion protein. Thus, said tag is capable of binding to one or more ligands, for example, one or more ligands of an affinity matrix such as a chromatography support or bead with high affinity. An example of said tag is a histidine tag (His-tag or HT), such as a tag comprising 6 residues of histidine (His6 or H6), which can bind to a column of nickel (Ni²⁺) or cobalt (Co²⁺) with high affinity.

His-tag has the desirable feature that it can bind its ligands under conditions that are denaturing to most proteins and disruptive to most protein-protein interactions. Thus, it can be used to remove the bait protein tagged with H6 following the disruption of protein-protein interactions with which the bait has participated.

Additional illustrative, non-limitative, examples of tags useful for isolating or purifying the conjugate or the polypeptide comprising SEQ ID NO: 1 or a variant thereof or a fusion protein include Arg-tag, FLAG-tag (DYKDDDDK; SEQ ID NO:59), Strep-tag (WSHPQFEK, SEQ ID NO:60), an epitope capable of being recognized by an antibody, such as c-myc-tag (recognized by an anti-c-myc antibody), HA tag (YPYDVPDYA, SEQ ID NO:61), V5 tag (GKPIPNPLLGLDST, SEQ ID NO:62), SBP-tag, S-tag, calmodulin binding peptide, cellulose binding domain, chitin binding domain, glutathione S-transferase-tag, maltose binding protein, NusA, TrxA, DsbA, Avi-tag, etc. (Terpe K., Appl. Microbiol. Biotechnol. 2003, 60:523-525), an amino acid sequence such as AHGHRP (SEQ ID NO:63) or PIHDHDHPHLVIHSGMTCXXC (SEQ ID NO:64), β-galactosidase and the like.

The tag can be used, if desired, for the isolation or purification of said fusion protein.

In another preferred embodiment, compound (i) of the invention is a polynucleotide encoding the polypeptide or the fusion protein disclosed above. In a preferred embodiment, compound (i) of the invention is a polynucleotide encoding a polypeptide comprising the sequence SEQ ID NO: 1 or a functionally equivalent variant thereof. In another embodiment, compound (i) of the invention is a polynucleotide encoding a conjugate comprising the polypeptide comprising the sequence SEQ ID NO: 1 or a functionally equivalent variant thereof and a chemical moiety that facilitates cellular uptake of the polypeptide or of the functionally equivalent variant thereof; more preferably is a polynucleotide encoding a fusion protein between the polypeptide comprising the sequence SEQ ID NO: 1 or a functionally equivalent variant thereof and a cell-penetrating peptide sequence.

The terms "polynucleotide", "nucleic acid" and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules. As used herein, mRNA refers to an RNA that can be translated in a cell.

In preferred embodiment, the polynucleotide of the invention is an mRNA.

mRNA can be chemically synthesized, can be obtained by means of *in vitro* transcription or can be synthesized *in vivo* in the target cell. The nucleotide sequences that form the polynucleotide encoding the conjugate or fusion protein of the invention are in the same correct reading frame for expression thereof.

In a preferred embodiment, component (i) of the combination of the invention is an mRNA encoding for a polypeptide consisting of the sequence SEQ ID NO: 1 or a polypeptide consisting of a functionally equivalent variant of SEQ ID NO: 1 or a polypeptide consisting of SEQ ID NO: 4.

In another embodiment, component (i) of the combination of the invention is a vector comprising a polynucleotide of the invention.

The term "vector", as used herein, refers to a nucleic acid sequence comprising the necessary sequences so that after transcribing and translating said sequences in a cell a polypeptide encoded by the polynucleotide of the invention is generated. Said sequence is operably linked to additional segments that provide for its autonomous replication in a host cell of interest. Preferably, the vector is an expression vector, which is defined as a vector which, in addition to the regions of the autonomous replication in a host cell, contains regions operably linked to the nucleic acid of the invention and which are capable of enhancing the expression of the products of the nucleic acid according to the invention. The vectors of the invention can be obtained by means of techniques widely known in the art.

Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. Suitable vectors comprising a polynucleotide of the invention are vectors derived from expression vectors in prokaryotes such as pUC18, pUC19, pBluescript and their derivatives, mp18, mp19, pBR322, pMB9, ColE1, pCRI, RP4, phages and "shuttle" vectors such as pSA3 and pAT28, expression vectors in yeasts such as vectors of the 2-micron plasmid type, integration plasmids, YEP vectors, centromeric plasmids and similar, expression vectors in insect cells such as the vectors of the pAC series and of the pVL series, expression vectors in plants such as vectors of the series pIBI, pEarleyGate, pAVA, pCAMBIA, pGSA, pGWB, pMDC, pMY, pORE and similar and expression vectors in superior eukaryote cells based on viral vectors (adenovirus, virus associated to adenovirus as well as retrovirus and, in particular, lentivirus) as well as non-viral vectors such as pSilencer 4.1-CMV (Ambion), pcDNA3, pcDNA3.1/hyg, pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCl, pSVL, pKSV-10, pBPV-1, pML2d and pTDT1. In a preferred embodiment, the polynucleotide of the invention is comprised in a vector selected from the group consisting of pEGFP or pBabe retroviral vectors and pTRIPZ or pSLIK lentiviral vectors.

The vector of the invention may be used to transform, transfect or infect cells that can be transformed, transfected or infected by said vector. Said cells may be prokaryotic or eukaryotic.

The vector preferably comprises the polynucleotide of the invention operationally bound to sequences that regulate the expression of the polynucleotide of the invention. The regulatory sequences of use in the present invention may be nuclear promoters or, alternatively, enhancer sequences and/or other regulatory sequences that increase expression of the heterologous nucleic acid sequence. In principle, any promoter can be used in the present invention provided said promoter is compatible with the cells wherein the polynucleotide is to be expressed. Thus, promoters suitable for realizing the present invention include, but are not necessarily limited to, constitutive promoters such as derivatives of eukaryotic virus genomes such as polyoma virus, adenovirus, SV40, CMV, avian sarcoma virus, hepatitis B virus, the metallothionein gene promoter, the herpes simplex virus thymidine kinase gene promoter, LTR regions of retroviruses, the immunoglobulin gene promoter, the actin gene promoter, the EF-1alpha gene promoter as well as inducible promoters wherein protein expression depends on the addition of a molecule or exogenous signal, such as tetracycline systems, the NPκB/UV light system, the Cre/Lox system and the heat shock genes promoter, the regulable RNA polymerase II promoters described in WO/2006/135436 and tissue-specific promoters.

In another embodiment, component (i) of the combination of the invention is a cell capable of secreting into the medium the polypeptide of the invention or the conjugate of the invention, preferably the polypeptide of the invention or the fusion protein of the invention.

Suitable cells capable of secreting a polypeptide of the invention include without limitation, cardiomyocytes, adipocytes, endothelial cells, epithelial cells, lymphocytes (B and T cells), mastocytes, eosinophils, vascular intima cells, primary cultures of isolated cells of different organs, preferably of cells isolated from Langerhans islets, hepatocytes, leukocytes, including mononuclear leukocytes, mesenchymal, umbilical cord or adult (of skin, lung, kidney and liver), osteoclasts, chondrocytes and other connective tissue cells. Cells of established lines such as Jurkat T cells, NIH-3T3, CHO, Cos, VERO, BHK, HeLa, COS, MDCK, 293, 3T3 cells, C2C12 myoblasts and W138 cells are also suitable. Persons skilled in the art will appreciate that the cells capable of secreting into the medium a polypeptide of the invention may be found forming microparticles or microcapsules so that the cells have a greater useful life in patients. Materials suitable for the formation of microparticles object of the invention include any biocompatible polymeric material which permits continuous secretion of the therapeutic products and which acts as support of the cells. Thus, said biocompatible polymeric material may be, for example, thermoplastic polymers or hydrogen polymers. Among the thermoplastic polymers we have acrylic acid, acrylamide, 2-aminoethyl methacrylate, poly(tetrafluoroethylene-cohexafluorpropylene), methacrylic-(7-cumaroxy) ethyl ester acid, N-isopropyl acrylamide, polyacrylic acid, polyacrylamide, polyamidoamine, poly(amino)-p-xylylene, poly(chloroethylvinylether), polycaprolactone, poly(caprolactone-co-trimethylene carbonate), polycarbonate urea) urethane, poly(carbonate) urethane, polyethylene, polyethylene and acrylamide copolymer, polyethylene glycol, polyethylene glycol methacrylate, poly(ethylene terephthalate), poly(4-hydroxybutyl acrylate), poly(hydroxyethyl methacrylate), poly(N-2-hydroxypropyl methacrylate), poly(lactic glycolic acid), poly(L-lactic acid), poly(gamma-methyl, Lglutamate), poly(methylmethacrylate), polypropylene fumarate), polypropylene oxide), polypyrrole, polystyrene, poly(tetrafluoroethylene), polyurethane, polyvinyl alcohol, polyethylene of ultra-high molecular weight, 6-(p-vinylbenzamide)-hexanoic acid N-pvinybenzyl-D-maltonamide and copolymers containing more than one of said polymers. Among the polymers of hydrogel type we have natural materials of alginate, agarose, collagen, starch, hyaluronic acid, bovine serum albumin, cellulose and their derivatives, pectin, chondroitin sulphate, fibrin and fibroin, as well as synthetic hydrogels such as Sepharose^{®} and Sephadex^{®}.

### Compound (ii) of the combination of the invention

Compound (ii) of the combination of the invention is a KRAS inhibitor.

The RAS proteins are members of the small GTPase protein family and serve as a binary switch in the signal transduction of most growth factor receptors including EGFRs, MET or KIT. RAS subfamily comprises four proteins encoded by three different genes: KRAS, HRAS and NRAS.

"KRAS" (or GTPase KRas, E.C. 3.6.5.2), as used herein, refers to the protein coded by Kirsten rat sarcoma 2 viral oncogene homolog, also known as "C-K-RAS", "K-RAS2A", "K-RAS2B", "K-RAS4A", "K-RAS4B", "KI-RAS", "KRAS1", "KRAS2", "K-ras", "KRAS proto-oncogene, GTPase", "c-Ki-ras", "K-Ras 2", "K-Ras", "Kirsten RAt Sarcoma virus", or "Kirsten Rat Sarcoma virus". The *KRAS* gene is an oncogene that encodes a small GTPase transductor protein called KRAS, which convert the nucleotide guanosine triphosphate (GTP) into guanosine diphosphate (GDP). The balance between nucleotide hydrolysis and exchange determines the levels of active KRAS in cells. Bound to GDP, KRAS is in an "OFF" state. Upon GDP to GTP exchange, usually in response to growth factors and facilitated by guanine-nucleotide exchange factors (GEF) such as SOS1/SOS2, KRAS cycles to its activated "ON" state. In this form, KRAS activates effector pathways, including the MAPK and PI3K pathways, to promote cellular proliferation and survival. KRAS returns to the OFF state when GTP is hydrolyzed to GDP, a process that is catalyzed by GTPase activating proteins (GAP) such as NF1. In its oncogenic form, KRAS remains predominantly in the active ON state as GTP hydrolysis, by its intrinsic GTPase function and enzymes, such as GAPs, is impaired.

KRAS gene was identified as a homologue of the Kirsten rat sarcoma virus responsible for malignant transformation of rodent cells. The human KRAS gene (Gene ID: 3845) is located on chromosome 12p12.1 encoded by 6 exons.

The sequence of the KRAS protein in humans corresponds to the sequence P01116 in the UniProt Database (version 255 of the entry as of 3 May 2023). There are two isoforms produced by alternative splicing: P01116-1 and P01116-2, which differ in the C-terminal region which is encoded by two alternative exons (IVA and IVB). The isoform P01116-1 has been chosen as the canonical sequence and is also known as 2A or K-Ras4A. The isoform P01116-2 is also known as 2B or K-Ras4B.

KRAS is the most frequently mutated oncogene in humans: more than 80% of pancreatic cancers, more than 30% of colorectal, cholangial cancers and lung adenocarcinomas harbor activating mutations of the KRAS gene.

Common activating mutations in the KRAS gene are KRAS-wild type amplification, a mutation in the KRAS gene that is a mutation at the glycine at position 12, at the glycine at position 13, or at the glutamine at position 61. In a more preferred embodiment, the mutation is selected from the group consisting of the G12S mutation, the G12V mutation, the G12D mutation, the G13D mutation, the G12C mutation, the G12R mutation, the G12F mutation, the G12I mutation, the G12A mutation, the G13C mutation, the G13R mutation, or the Q61L mutation. In a preferred embodiment the mutation is G12C mutation. In a preferred embodiment, the mutation is the G12D mutation.

"KRAS inhibitor", as used herein, refers to any compound capable of causing a decrease in the activity of one or more KRAS proteins, preferably of mutant KRAS proteins, including those compounds which prevent expression of KRAS gene, and compounds which lead to reduced KRAS mRNA or protein levels. The term "KRAS inhibitor", as used herein, refers to compounds that inhibit the catalytic activity of KRAS enzyme by direct binding to KRAS or KRAS mutants, either covalently or reversibly; and also refers to compounds that inhibit upstream activators of KRAS or that target the KRAS interaction with said upstream activators of KRAS, thereby inhibiting signaling pathways downstream from KRAS.

In a preferred embodiment, the KRAS inhibitors can be direct inhibitors or indirect inhibitors. Direct KRAS inhibitors are those that directly inhibit the KRAS proteins, preferably the mutant KRAS proteins, by binding to them, either reversibly or irreversibly. Indirect KRAS inhibitors are those that act indirectly and that do not bind to the KRAS proteins, preferably KRAS mutant proteins, acting on the crucial steps required for KRAS activation, for instance by targeting and inhibiting KRAS interaction with associated proteins.

The expression of a protein or nucleic acid is considered reduced when its levels decrease with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

The reference value refers to the level of protein or nucleic acid in control subject, which may be a subject who does not suffer a specific disease.

Suitable methods for determining whether an inhibitor is capable of decreasing KRAS mRNA levels include, without limitation, standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence *in situ* hybridization (FISH), including variants such as Flow-FISH, qFiSH and double fusion FISH (D-FISH), and the like. Preferably quantitative or semi-quantitative RT-PCR is preferred. Real-time quantitative or semi-quantitative RT-PCR is particularly advantageous.

The nucleic acid contained in the sample (e.g., cell or tissue prepared from the subject) is first extracted according to standard methods, for example using lytic enzymes or chemical solutions or extracted by nucleic-acid-binding resins following the manufacturer's instructions.

In case the mRNA is measured in a biological sample, said biological sample may be treated to physically, mechanically or chemically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor. In a particular embodiment samples can be obtained from fresh tumor tissue or from OCT embedded frozen tissue.

In order to normalize the values of mRNA expression among the different samples, it is possible to compare the expression levels of the mRNA of interest in the test samples with the expression of a control RNA. A "control RNA", as used herein, relates to RNA whose expression levels do not change or change only in limited amounts in tumor cells with respect to non-tumorigenic cells. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, IPO8, HPRT, GAPDH, PSMB4, tubulin and β-actin.

The relative gene expression quantification may be calculated according to the comparative threshold cycle (Ct) method using a housekeeping gene as an endogenous control and commercial RNA controls as calibrators. Final results are determined according to the formula 2^{-(ΔCt sample-ΔCt calibrator)}, where ΔCt values of the calibrator and sample are determined by subtracting the Ct value of the target gene from the value of the control gene.

Suitable methods for determining whether an inhibitor acts by decreasing the KRAS protein levels, include the quantification by means of conventional methods, for example, using antibodies with a capacity to specifically bind to the proteins encoded by KRAS genes (or to fragments thereof containing antigenic determinants) and subsequent quantification of the resulting antibody-antigen complexes.

The antibodies to be employed in these assays can be, for example, polyclonal sera, hybridoma supernatants or monoclonal antibodies, antibody fragments, Fv, Fab, Fab' and F(ab')2, ScFv, diabodies, triabodies, tetrabodies and humanized antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

On the other hand, the determination of the levels of the KRAS proteins, can be carried out by constructing a tissue microarray (TMA) containing the subject samples assembled, and determining the expression levels of the corresponding protein by immunohistochemistry techniques. Immunostaining intensity can be evaluated by two or more different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumor cells and the specific cut-off for each marker. As a general criterion, the cut-offs are selected in order to facilitate reproducibility, and when possible, to translate biological events. Alternatively, the immunostaining intensity can be evaluated by using imaging techniques and automated methods such as those disclosed in Rojo, M.G. et al. (Folia Histochem. Cytobiol. 2009; 47: 349-54) or Mulrane, L. et al. (Expert Rev. Mol. Diagn. 2008; 8: 707-25).

Alternatively, in another particular embodiment, the levels of the KRAS proteins are determined by Western blot. Western blot is based on the detection of proteins previously resolved by gel electrophoreses under denaturing conditions and immobilized on a membrane, generally nitrocellulose, by the incubation with an antibody specific and a developing system (e.g. chemoluminiscent).

A KRAS inhibitor may inhibit KRAS activity by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or even 100% and all ranges between 5% and 100%. Suitable methods for determining whether an inhibitor acts by decreasing the KRAS activity include any method which allows detecting a decrease in the phosphorylation of a key downstream effector of KRAS, such as the extracellular signal-regulated kinase (ERK). Methods for detecting KRAS activity are known in the art and include without limitation active KRAS pulldown experiments, Ras binding domain (RBD) pulldown; western blot, immunoblot, immunoassays and immunofluorescence to detect phosphorylation state of KRAS downstream effectors, nucleotide exchange, transcriptomic assays, detection of protein-protein interactions (for example but not limited to KRAS-CRAF) (Canon J. et al. 2019. Nature, 575:217-223; Kim D. et al. 2023 May 31. Nature, doi: 10.1038/s41586-023-06123-3. Epub ahead of print. PMID: 37258666; Hallin J. et al. 2020. Cancer Discov, 10(1):54-71). Assays to determine the activity of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration.

KRAS inhibitors can be any organic or inorganic molecule, including modified and unmodified nucleic acids such as antisense nucleic acids, RNA interference (RNAi) agents such as siRNA, shRNA or miRNA, peptides, proteins, peptidomimetics, receptors, ligands, antibodies and small organic molecules that are useful in treating cancer.

"Small chemical compound", as used herein, relates to a molecule that regulates a biological process, in the present case inhibits catalytic activity of KRAS. This compound can be natural or artificial.

In a preferred embodiment, KRAS inhibitors useful in the present invention are selected from Table 1.

| TABLE 1: KRAS INHIBITORS SUITABLE FOR USE ACCORDING TO THE INVENTION | |
|---|---|
| I | Mutant-specific KRAS inhibitors that target KRAS G12C: |
| | Sotorasib /AMG 510 (Amgen), Adagrasib /MRTX849 (Mirati), D-1553 (InventisBio), JDQ443 (Novartis), RG6330/GDC-6036 (Roche), LY3537982 (Eli Lilly), BI 1823911 (Boehringer Ingelheim), JAB-21822 (Jacobio), GFH925 (GenFleet), GH35 (Genhouse Bio), RMC-6291 (Revolution Medicines) |
| II | Mutant-specific KRAS inhibitors that target KRAS G12D: |
| | MRTX1133 (Mirati), BI-KRASG12D1-3 (Boehringer Ingelheim), RAS(ON) G12D (Revolution Medicines) |
| III | Mutant-specific KRAS inhibitors that target KRAS G13C: |
| | RAS(ON) G13C (Revolution Medicines) |
| IV | Direct Pan-KRAS inhibitors: |
| | RSC-1255 (RasCal Therapeutics), BI-pan-KRAS1-4 inhibitors (Boehringer Ingelheim), BI-pan-KRASdegrader1 (Boehringer Ingelheim), RMC-6236 (Revolution Medicines) |
| V | Indirect Pan-KRAS SHP2 inhibitors: |
| | TNO155 (Novartis), RMC-4630 (Revolution Medicines), JAB-3068 (Jacobio), JAB-3312 (Jacobio), RG-6433/RLY-1971 (Roche/Genentech), Clinical BBP-398 (BridgeBio/Navire), ERAS-601 (Erasca), PF-07284892/ARRY-558 (Pfizer), SH3809 (Nanjing Sanhome) |
| VI | Indirect Pan-KRAS SOS1 inhibitors: |
| | BI 1701963 (Boehringer Ingelheim), RMC-5845 (Revolution Medicines), BAY-293 (Bayer), SDGR5 (Schrödinger/BMS), GH52 (Genhouse Bio), ERAS-9 (Erasca), SOS1i (Mirati), BI-3406 (Boehringer Ingelheim) |
| VII | An interference RNA specific for the KRAS sequence |
| VIII | An antisense oligonucleotide specific for the KRAS sequence |
| IX | A ribozyme or DNA enzyme specific for the KRAS sequence |
| X | Inhibitory antibodies which bind specifically to KRAS and inhibit KRAS activity |
| XI | Aptamers and spiegelmers |

In a preferred embodiment the KRAS inhibitor is a mutant-specific KRAS inhibitor. A "mutant-specific KRAS inhibitor" or a "mutant-selective KRAS inhibitor" or an "allele-selective KRAS inhibitor" or an "allele-specific KRAS inhibitor" is a KRAS inhibitor that targets individual variant alleles of KRAS.

In a preferred embodiment the KRAS inhibitor is a mutant-specific KRAS inhibitor that targets KRAS G12C. These compounds covalently bind to cysteine 12 in GDP-KRAS G12C, acting as inactive state-selective KRAS drugs, and are thus direct irreversible inhibitors. In a more preferred embodiment, the KRAS inhibitor is selected from the group consisting of compounds listed in item I of Table I or a pharmaceutically acceptable salt thereof; more preferably is selected from sotorasib and adagrasib.

In a preferred embodiment the KRAS inhibitor is a mutant-specific KRAS inhibitor that targets KRAS G12D. MRTX1133 and BI-KRASG12D1-3 can reversibly directly bind to KRAS G12D. In a more preferred embodiment, the KRAS inhibitor is selected from the group consisting of compounds listed in item II of Table 1 or a pharmaceutically acceptable salt thereof; more preferably is MRTX1133.

In a preferred embodiment the KRAS inhibitor is a mutant-specific KRAS inhibitor that targets KRAS G13C. These inhibitors are direct inhibitors that bind this allele. In a more preferred embodiment, the KRAS inhibitor is selected from the group consisting of compounds listed in item III of Table 1 or a pharmaceutically acceptable salt thereof; more preferably is RAS(ON) G13C.

In a preferred embodiment the inhibitor is a KRAS G12C inhibitor. In another preferred embodiment the inhibitor is a KRAS G12D inhibitor.

Mutant-selective KRAS G12C inhibitors target a fraction (approximately 13.6%) of all KRAS-driven cancers. A broad arsenal of KRAS drugs is needed to comprehensively conquer KRAS-driven cancers.

In a preferred embodiment the KRAS inhibitor is a Pan-KRAS inhibitor.

The term "Pan-KRAS inhibitor", as used herein, refers to a KRAS inhibitor that targets a broad range of KRAS alterations and that do not discriminate between KRAS mutants. Therefore, Pan-Kras inhibitors are able to target the KRAS pathway regardless of the specific KRAS mutation. Pan-KRAS inhibitors have the potential to address broad patient populations, including KRAS G12D, KRAS G12V, KRAS G13D, KRAS G12R, and KRASG12A mutants or KRAS wild-type amplified cancers, as well as cancers with acquired resistance to KRAS G12C inhibitors.

Pan-KRAS inhibitors can be direct pan-KRAS inhibitors or indirect pan-KRAS inhibitors.

Direct Pan-KRAS inhibitors are compounds which act by forming a bond with one or more KRAS proteins or with the mutated amino acid in KRAS proteins, thereby locking KRAS in the inactive state.

Direct pan-KRAS inhibitors are reported to show activity against a broad range of KRAS-driven cell lines, including KRASG12C, KRASG12D, KRASG12V, and KRASG13D-driven cells, whereas HRAS- and NRAS-mutated cell lines did not exhibit sensitivity (Hofmann M.H. et al. Expanding the reach of precision oncology by drugging all KRAS mutants. 2022. Cancer Discov, 12(4): 924-937).

Exemplary, non-limitative direct Pan-KRAS inhibitors are: RSC-1255, targeting Pan-RAS; BI-Pan-KRAS1-4 inhibitors, targeting Pan-KRAS, KRAS G12D/V, and KRAS wild-type; BI-pan-KRASdegrader1, targeting Pan-KRAS, KRAS G12C/D/V/A, KRAS G13C, KRAS A146T/P, KRAS Q61E/P, and KRAS wild-type; RMC-6236, targeting Pan-RAS, KRAS G12D/V, KRAS G13D, KRAS Q61K, and RAS wild-type.

In a preferred embodiment the KRAS inhibitor is a direct Pan-KRAS inhibitor. In a more preferred embodiment, the KRAS inhibitor is selected from the group consisting of compounds listed in item IV of Table 1 or a pharmaceutically acceptable salt thereof.

Indirect Pan-KRAS inhibitors are pathway inhibitors that inhibit upstream activators of KRAS. Preferably, indirect Pan-KRAS inhibitors interfere with KRAS nucleotide exchange and activation by inhibiting SHP2 or the GEF SOS1.

In a preferred embodiment, the KRAS inhibitor is an indirect Pan-KRAS SHP2 inhibitor. SHP2 inhibitors stabilize the auto-inhibited conformation of the enzyme and thereby disrupt SOS1-mediated nucleotide exchange of KRAS. In a more preferred embodiment, the KRAS inhibitor is selected from the group consisting of compounds listed in item V of Table 1 or a pharmaceutically acceptable salt thereof.

A "SHP2" inhibitor, as used herein, refers to any compound capable of causing a decrease in the activity of SHP2, including those compounds which prevent expression of SHP2 gene, and compounds which lead to reduced SHP2 mRNA or protein levels. SHP2 inhibitors primarily inhibit the catalytic activity of SHP2 enzymes.

"SHP2", as used herein refers to tyrosine-protein phosphatase non-receptor type 11 (PTN11), also known as protein-tyrosine phosphatase 1D (PTP-1D), Src homology region 2 domain-containing phosphatase-2 (SHP2) or protein-tyrosine phosphatase 2C (PTP-2C). The sequence of SHP2 in humans corresponds to the sequence Q06124 in the UniProt database (version 255 of the entry as of 3 May 2023).

In a more preferred embodiment the KRAS inhibitor is an indirect Pan-KRAS SOS1 inhibitor. Inhibitors of the GEF SOS1 block the interaction of SOS1 with KRAS-GDP, preventing nucleotide exchange and GTP loading of KRAS. In a more preferred embodiment, the KRAS inhibitor is selected from the group consisting of compounds listed in item VI of Table 1 or a pharmaceutically acceptable salt thereof; preferably are selected from the group consisting of BAY-293 and BI-3406.

A "SOS1 inhibitor", as used herein, refers to any compound capable of causing a decrease in the activity of SOS1, including those compounds which prevent expression of SOS1 gene, and compounds which lead to reduced SOS1 mRNA or protein levels. SOS1 inhibitors primarily inhibit the catalytic activity of SOS1 enzymes.

"SOS1", as used herein, refers to Son of sevenless homolog 1. The sequence of SOS1 protein in humans corresponds to the sequence Q07889 (version 238 of the entry as of 3 May 2023).

In a preferred embodiment, the KRAS inhibitor is selected from the group consisting of sotorasib, adagrasib, MRTX1133, BAY-293 and BI-3406; preferably is selected from the group consisting of sotorasib, adagrasib, and MRTX1133.

All the compounds listed in items I to VI of Table 1 also include pharmaceutically acceptable salts, solvates, polymorphs or cocrystals thereof. Prodrugs of these compounds are also included.

In a more preferred embodiment the KRAS inhibitor is selected from the group consisting of compounds listed in items I, II, III, IV, V and VI of Table 1 or a pharmaceutically acceptable salt thereof; preferably compounds listed in items I, II, III, IV, V and VI of Table 1; more preferably compounds listed in items I, II, III and IV of Table 1; even more preferably compounds listed in items I, II and III of Table 1.

The term "pharmaceutically acceptable" refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

The term "pharmaceutically acceptable salt" embraces salts with a pharmaceutically acceptable acid or base. Pharmaceutically acceptable acids include both inorganic acids, for example and without limitation hydrochloric, sulfuric, phosphoric, diphosphoric, hydrobromic, hydroiodic and nitric acid and organic acids, for example and without limitation citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example and without limitation alkyl amines, arylalkyl amines and heterocyclic amines.

The term "solvate" according to this invention is to be understood as meaning any solid form of the above compounds which has another molecule attached to it via non-covalent bonding. Examples of solvates include hydrates and alcoholates, preferably C1-C6 alcoholates, e.g. methanolate.

The term "polymorph" according to this invention is to be understood as a specific crystalline form of a compound that can crystallize in different forms.

The term "cocrystal", as used herein, is to be understood as a crystalline structure composed of a KRAS inhibitor and at least another component. As used herein, the term "interference RNA" or "iRNA" refers to RNA molecules capable of silencing the expression of one or more KRAS proteins, particularly KRAS mutant proteins or of any gene needed for KRAS function. To that end, iRNA are typically double-stranded oligonucleotides having at least 30 base pairs in length, and they more preferably comprise about 25, 24, 23, 22, 21, 20, 19, 18 or 17 ribonucleic acid base pairs. Several different types of molecules have been used effectively in iRNA technology including small interfering RNA (siRNA) sometimes known as short interference RNA or silencer RNA, micro RNA (miRNA) which normally differs from siRNA because they are processed from single-stranded RNA precursors and they are shown to be only partially complementary to the target mRNA and short hairpin RNA (shRNA).

Small interfering RNA (siRNA) agents are capable of inhibiting target gene expression by interfering RNA. siRNAs may be chemically synthesized, or may be obtained by *in vitro* transcription, or may be synthesized *in vivo* in target cell. Typically, siRNAs consist of a double-stranded RNA from 15 to 40 nucleotides in length and may contain a protuberant region 3' and/or 5' from 1 to 6 nucleotides in length. Length of protuberant region is independent from total length of siRNA molecule, siRNAs act by post-transcriptional degradation or silencing of target messenger.

siRNA may be denominated shRNA (short hairpin RNA) characterized because the antiparallel strands that form siRNA are connected by a loop or hairpin region. siRNAs are constituted by a short antisense sequence (19 to 25 nucleotides) followed by a loop of 5-9 nucleotides, and the sense strand. shRNAs may be encoded by plasmids or virus, particularly retrovirus and, more particularly, retrovirus and under the control of promoters such as U6 promoter for RNA polymerase III.

The siRNAs for use within the context of the invention are substantially homologous to KRAS mRNA, particularly to mutant KRAS mRNA, or its protein-coding genome sequence. The term "substantially homologous" is understood to mean that siRNAs have a sequence sufficiently complementary or similar to target mRNA so that siRNA may be able to provoke mRNA degradation by RNA interference. Suitable siRNAs to provoke interference include siRNAs formed by RNA, as well as siRNAs containing chemically different modifications such as:
- siRNAs in which the links between nucleotides are different from those appearing in nature, such as phosphorothioate links;
- stranded-RNA conjugates with a functional reagent, such as a fluorophoro;
- modification of the ends of RNA strands, particularly the 3' end by the combination with different functional hydroxyl groups at 2'-position;
- sugar-modified nucleotides such as O-alkylated radicals at 2'-position such as 2'-O-methylribose or 2'-O-fluororibose;
- base-modified nucleotides such as halogenated bases (for example 5-bromouracil and 5-iodouracil), alkylated bases (for example 7-methyl-guanosine).

The siRNAs and shRNAs for use within the context of the invention may be obtained using a series of techniques known to a person skilled in the art. For example, siRNA may be chemically synthesized from protected ribonucleoside phosphoramidites in a conventional DNA/RNA synthesizer. Alternatively, siRNA may be produced by recombinant dicer from plasmid and viral vectors, where the coding region of siRNA strand or strands is under operative control of RNA polymerase III promoters. RNase Dicer processes shRNA into siRNA in cells.

The KRAS region which is taken as a basis for the design of siRNA is not limitative and may contain a region of coding sequence (between the initiation codon and the termination codon) or, alternatively, may contain sequences from the 5' or 3' untranslated region, preferably from 25 to 50 nucleotides in length and in any position in 3' position with regard to the initiation codon. A procedure for siRNA design involves the identification of sequence motif AA(N19)TT wherein N may be any nucleotide in KRAS sequence and the selection of those that exhibit a high content in G/C. If said sequence motif is not found, it is possible to identify sequence motif NA(N21) wherein N may be any nucleotide.

In a preferred embodiment the KRAS inhibitor is an siRNA. In a more preferred embodiment the siRNA is a commercial siRNA from Santa Cruz Biotechnology, particularly sc-35731.

In another embodiment, the inhibitor is an "antisense oligonucleotide" specific to KRAS, i.e., molecules whose sequence is complementary to mRNA coding for KRAS, i.e., complementary to cDNA coding chain. The antisense oligonucleotide may be complementary to a complete coding region or a region of same including both the coding region and the 5' and 3' untranslated regions. The antisense oligonucleotides may consist of 5, 10, 15, 20, 25, 30, 35, 40, 45, 50 or more nucleotides in length. The antisense oligonucleotides may be obtained by chemical synthesis or by enzymatic binding reactions widely known to a person skilled in the art. For example, an antisense oligonucleotide may further contain modified nucleotides which increase its biological stability or the stability of the bicatenary DNA-RNA complexes formed between the antisense oligonucleotide and the target polynucleotide, such as phosphorothioate derivatives, peptide nucleic acids and acridine-substituted oligonucleotides. Modified oligonucleotides that may be used for the preparation of antisense nucleic acid include 5-fluorouracil, 5-bromouracil, 5-chlorouracil, 5-iodouracil, hypoxanthine, xanthine, 4-acetyl-citosine, 5-(carboxyhydroxylmethyl) uracil, 5-carboxymethylaminomethyl-2-thiouridine, 5-carboxymethyl-aminomethyl uracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcitosine, 5-methylcitosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6- isopentenyladenine, uracil-5-oxyacetic acid, pseudouracil, queosine, 2- thiocitosine, 5-methyl-2-thiouracil, 2-thiouracil, 4-thiouracil, 5-methyluracil, uracil-5-oxyacetic acid methyl ester, 5-methyl-2-thiouracil, 3-(3-amino-3-N-2-carboxypropyl)uracil, and 2,6- diaminopurine. Alternatively, the antisense nucleic acid may be produced biologically using an expression vector in which the antisense-oriented nucleic acid has been cloned.

Another group of compounds that may form part of the present invention are catalytically active nucleic acids known as ribozimes. "Ribozimes" comprise a catalytic region and a second region whose sequence is complementary to target nucleic acid and confers substrate specificity on the ribozime. After the interaction between the ribozime and its substrate by hybridization and coupling between complementary regions of target nucleic acid and ribozime, an activation of the catalytic region is produced provoking the inter- or intramolecular rupture of target nucleic acid. Basic considerations for the design of ribozimes are widely known to a person skilled in the art (see, for example, Doherty and Doudna (Annu. Ref. Biophys. Biomolstruct. 2000; 30:457- 75).

Another type of compounds that may form part of the compositions of the invention include inhibitory antibodies. The term "inhibitory antibody" is understood to mean, according to the present invention, an antibody that binds to any KRAS protein, preferably to mutant KRAS proteins, provoking the inhibition of its catalytic activity.

Antibodies may be prepared using any method known to a person skilled in the art. Thus, polyclonal antibodies are prepared by immunization of an animal with the protein aimed to be inhibited. Monoclonal antibodies can be prepared using the method described by Kohler, Milstein et al (Nature, 1975, 256: 495). Once antibodies capable of binding to KRAS proteins are identified, those antibodies capable of inhibiting KRAS activity using the abovementioned assays for determination of KRAS activity will be selected, particularly KRAS mutant activity. Suitable antibodies in the present invention include intact antibodies which comprise an antigen-binding variable region and a constant region, fragments "Fab", "F(ab')2" y "Fab'", Fv, scFv, diabodies and bispecific antibodies.

Other compounds capable of inhibiting KRAS expression that may form part of the compositions of the invention include aptamers and spiegelmers. "Aptamers and spiegelmers" are single-stranded or double-stranded D- or L-nucleic acids that specifically bind to the protein resulting in a modification of the biological activity of protein (KRAS proteins, particularly mutant KRAS proteins). Aptamers and spiegelmers are 15 to 80 nucleotides in length and, preferably, 20 to 50 nucleotides in length.

In an embodiment, the combination of the invention is a conjugate between component (i) and component (ii) of the combination of the invention, particularly is a conjugate between a polypeptide comprising the sequence SEQ ID NO: 1 or a functionally equivalent variant thereof and a KRAS inhibitor.

In some embodiments, a conjugation between components (i) and (ii) are via a noncleavable linker. In some embodiments, a conjugation between components (i) and (ii) are via a cleavable linker. Exemplary non cleavable linkers and cleavable linkers are described in US8088387, US8142784, WO2013075048, US6630579, US8512707, US9120854, US9023351, US20160095938, US9446146, WO2005009369, US5773001, US6214345, US10111954, US8153768, US7829531, US20160082119, WO2018218004, US8568728, WO2015057699, US20170182181, US9198979, the content of each of which is incorporated herein by reference in its entirety.

In another aspect, the invention relates to a pharmaceutical composition comprising a pharmaceutically effective amount of a combination of the invention together with a pharmaceutically acceptable excipient.

As it is used in the present invention, the expression "pharmaceutical composition" relates to a formulation that has been adapted for administering a predetermined dose of one or several therapeutic useful agents to a cell, a group of cells, an organ, a tissue or an animal in which cell division is uncontrolled, such cancer.

The pharmaceutical composition of the invention contains a pharmaceutically effective amount of a combination according to the invention and a pharmaceutically active carrier. The pharmaceutical composition of the invention comprises a polypeptide comprising the sequence SEQ ID NO: 1, a functionally equivalent variant thereof, a conjugate according to the invention, a polynucleotide encoding the polypeptide or the conjugate, a vector comprising the polynucleotide or a cell capable of secreting into the medium the polypeptide or the conjugate and a KRAS inhibitor. Suitable functionally equivalent variants of the polypeptide of SEQ ID NO: 1, suitable conjugates, fusion proteins, polynucleotides, vectors or cells for use in the pharmaceutical composition according to the invention are as defined above.

The expression "pharmaceutically effective amount", as used herein, is understood as an amount capable of providing a therapeutic effect, and which can be determined by the person skilled in the art by commonly used means. The amount of the Omomyc polypeptide, of the functionally equivalent variant thereof, of the conjugate, fusion protein, polynucleotide, vector, cell or of the KRAS inhibitor that may be combined in the pharmaceutical compositions according to the invention will vary depending upon the subject and the particular mode of administration. Those skilled in the art will appreciate that dosages may also be determined with guidance from Goodman and Goldman's The Pharmacological Basis of Therapeutics, Ninth Edition (1996), Appendix II, pp. 1707-1711 and from Goodman and Goldman's The Pharmacological Basis of Therapeutics, Tenth Edition (2001), Appendix II, pp. 475-493.

The appropriate dosage of the active principle or principles within the pharmaceutical composition will depend on the type of cancer to be treated, the severity and course of the disease, whether the composition is administered for preventive or therapeutic purposes, previous therapy, the patient's clinical history and response to the peptide or polypeptide, and the discretion of the attending physician.

The amount of polypeptide comprising the sequence SEQ ID NO:1, the functionally equivalent variant thereof, the fusion protein, the conjugate, polynucleotide, vector or cell is suitably administered to the patient at one time or over a series of treatments. Depending on the type and severity of the disease, an appropriate dosage level will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day.

In a preferred embodiment, the amount of the first component is of about 3.75 mg/kg, of subject body weight per day, preferably administered four times per week, preferably intranasally administered. In a preferred embodiment, the amount of the first component is of about 8 to 15 mg/m², preferably of 10 to 12 mg/m², more preferably 11.25 mg/m² per day, preferably administered four times per week, preferably intranasally administered.

In a preferred embodiment, the amount of the first component is of about 50 mg/kg, of subject body weight per day, preferably administered twice per week, preferably intravenously administered. In a preferred embodiment, the amount of the first component is of about 100 to 200 mg/m², preferably of 125 to 175 mg/m², preferably of 140 to 160 mg/m², more preferably 150 mg/m² per day, preferably administered twice per week, preferably intravenously administered.

A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day.

In an embodiment, the combinations or compositions can be administered once a week, twice a week, three times a week, four times a week, five times a week, six times a week or seven times a week. In an embodiment, the combinations or compositions can be administered once a week. In another embodiment, the combinations or compositions can be administered twice per week. In another embodiment, the combinations or compositions can be administered four times a week. In another preferred embodiment, the first component of the combination or composition is administered four times a week and the second component of the combination or composition is administered once a week. In another embodiment, the first component of the combination or composition is administered twice per week and the second component of the combination or composition is administered once a week. Both compounds can be concomitantly administered or sequentially administered. When the compounds are sequentially administered, the administration of the first compound is discontinued before starting with the second compound.

The duration of the treatment can be at least one week, at least two weeks, at least three weeks, at least four weeks, at least five weeks, at least six weeks, at least seven weeks, at least eight weeks, at least nine weeks, at least ten weeks or more. Preferably, the duration of the treatment is at least four weeks. In another embodiment, the duration of the treatment is at least three weeks.

The amount of the KRAS inhibitor depends on the specific agent used and may be about 0.01 mg/kg to about 200 mg/kg, preferably 0.01 mg/kg to about 150 mg/kg, more preferably 0.01 mg/kg to about 100 mg/kg, preferably 0.01 mg/kg to about 75 mg/kg, 0.01 mg/kg to about 50 mg/kg, more preferably 0.5 mg/kg to about 50 mg/kg, preferably from about 1 mg/kg to about 25 mg/kg, even more preferably from about 3 mg/kg to about 20 mg/kg, more preferably from about 5 mg/kg to about 20 mg/kg, more preferably from about 8 mg/kg to about 18 mg/kg, more preferably from about 10 mg/kg to 18 mg/kg of subject body weight per day, one or more times a day, to obtain the desired therapeutic effect, preferably administered orally. In a preferred embodiment, the amount of the KRAS inhibitor is of about 2.5 mg/kg, of subject body weight per day or 7.5 mg/m² per day, preferably administered once a week, more preferably administered daily, more preferably administered orally. In a preferred embodiment, the amount of the KRAS inhibitor is of about 0.5 mg/kg, of subject body weight per day or 1.5 mg/m² per day, preferably administered once a week, more preferably administered daily, more preferably administered orally. In a preferred embodiment, the amount of the KRAS inhibitor is of about 5 mg/kg, of subject body weight per day or 15 mg/m² per day, preferably administered once a week, more preferably administered daily, more preferably administered orally. In another preferred embodiment, the amount of the KRAS inhibitor is of about 10 mg/kg, of subject body weight per day, or 30 mg/m² per day, preferably administered once a week, more preferably administered daily, more preferably administered orally. In another preferred embodiment, the amount of the KRAS inhibitor is of about 50 mg/kg, of subject body weight per day, or 150 mg/m² per day, preferably administered once a week, more preferably administered daily, more preferably administered orally. In another preferred embodiment, the amount of the KRAS inhibitor is of about 100 mg/kg, of subject body weigh per day, or 300 mg/m² per day, preferably administered once a week, more preferably administered daily, more preferably administered orally. The KRAS inhibitor is preferably administered 7 days a week, preferably during 4 weeks.

The pharmaceutical compositions according to the invention, which contain a first component (i) selected from a polypeptide comprising SEQ ID NO:1, a functionally equivalent variant thereof, a fusion protein, a conjugate, a polynucleotide, a vector or a cell according to the invention and a second component (ii) which is a KRAS inhibitor, may be presented as a single formulation (for example, as a tablet or a capsule comprising a fixed quantity of each one of the components) or can, on the other hand, be presented as separate formulations to be later combined for joint, sequential, or separate administration. The compositions of the invention also include the formulation as a kit-of-parts wherein the components are formulated separately but are packaged in the same container. Those skilled in the art will appreciate that the formulation of the different components in the pharmaceutical composition according to the invention may be similar, in other words, similarly formulated (in tablets or pills), which allows their administration by the same route. In the case where the different components of the invention are formulated separately, the two components can be presented in a blister. Each blister contains the drugs that must be consumed during the day. If the drugs must be administered several times a day, the drugs corresponding to each administration can be placed in different sections of the blister, preferably recording in each section of the blister the time of day when they should be administered. Alternatively, the components of the composition of the invention can be formulated differently so that the different components are differently administered. Thus, it is possible that the first component is formulated for its intravenous administration and the second component is formulated as a tablet or capsule for its oral administration or vice versa. The ratio between the components that are part of the combinations or pharmaceutical compositions according to the invention can be adjusted by the skilled person depending on the antitumor agent used in each particular case, as well as of the desired indication. Thus, the invention envisages compositions wherein the ratio between the quantities of component (i) and component (ii) can range from 50:1 to 1:50, in particular from 40:1 to 1:40, in particular from 30:1 to 1:30, in particular from 20:1 to 1:20, from 1:10 to 10:1, or from 5:1 to 1:5. In a more particular embodiment, the ratio between quantities ranges from 1:1 to 1:5, preferably from 1:1 to 1:3. In a more preferred embodiment, the ratio ranges from 1:1 to 1:1.5, preferably from 1:1.3 to 1:1.4, more preferably 1:1.34. In another preferred embodiment, the ratio ranges from 1:1 to 1:2.8, preferably from 1:2.6 to 1:2.7, more preferably 1:2.67. In another particular embodiment, the ratio between quantities ranges from 30:1 to 5:1, preferably from 30:1 to 8:1, more preferably from 25:1 to 15:1, more preferably from 20:1 to 10:1. In a preferred embodiment the ratio between quantities ranges from 20:1 to 1:20. In an embodiment, the ratio is 20:1. In another embodiment the ratio is 1:20. In another embodiment, the ratio is 10:1. Exceptionally good results have been obtained when the ratio of component (i):component (ii), more preferably the ratio of Omomyc:sotorasib, Omomyc:adagrasib, Omomyc:MRTX1133, Omomyc:BAY-293, or Omomyc:BI-3406, ranges from 1:1 to 2,000,000:1; preferably from 2:1 to 2,000,000:1; preferably from 3:1 to 2,000,000:1; preferably from 3:1 to 1,905,136:1; more preferably from 6:1 to 1,000,000:1; more preferably from 8:1 to 800,000:1; more preferably from 24:1 to 500,000:1; more preferably from 29:1 to 500,000:1; more preferably from 36:1 to 100,000:1; more preferably from 48:1 to 50,000:1; more preferably from 143:1 to 10,000:1; more preferably from 268:1 to 5,000:1; more preferably from 889:1 to 1,000:1.

In a preferred embodiment, the ratio of component (i):component (ii), more preferably the ratio of Omomyc:sotorasib, ranges from 8:1 to 2,000,000:1; preferably from 8:1 to 1,905,136:1; more preferably from 8:1 to 1,900,000:1; even more preferably from 29:1 to 245,824:1; even more preferably from 29:1 to 240,000:1; even more preferably from 3,969:1 to 245,824:1; even more preferably from 4000:1 to 245,000:1; even more preferably from 7,682:1 to 127,009:1; even more preferably from 8,000:1 to 120,000:1. Although these ratios are valid for treating any kind of cancer, more preferably, these ratios are obtained when a cancer selected from the group consisting of non-small cell lung cancer or colorectal cancer are treated, even more preferably when the cancer is mutated in KRAS, more preferably having a KRAS G12C mutation. In a preferred embodiment, the ratio of component (i):component (ii), more preferably the ratio of Omomyc:sotorasib, ranges from 25:1 to 300,000:1 in non-small cell lung cancer, preferably having a KRAS mutation, more preferably having a KRAS G12C mutation, more preferably from 29:1 to 245,824:1; preferably from 29:1 to 240,000:1, preferably from 3,500:1 to 250,000:1; preferably from 3,969:1 to 245,824:1; preferably from 4,000:1 to 240,000:1; preferably from 25:1 to 120:1; preferably from 29:1 to 114:1. In a preferred embodiment, the ratio of component (i):component (ii), more preferably the ratio of Omomyc:sotorasib, ranges from 7,000:1 to 130,000:1 in colorectal cancer, preferably having a KRAS mutation, more preferably having a KRAS G12C mutation, more preferably from 7,500:1 to 128,000:1; more preferably from 7,682:1 to 127,009:1; more preferably from 8,000:1 to 125,000:1.

In a preferred embodiment, the ratio of component (i):component (ii), more preferably the ratio of Omomyc:adagrasib, ranges from 30:1 to 2,000,000:1; more preferably from 35:1 to 1,900,000:1; more preferably from 36:1 to 1,813.190:1; even more preferably from 38:1 to 1,500,000:1; even more preferably from 40:1 to 1,500.000:1. Although these ratios are valid for treating any kind of cancer, more preferably, these ratios are obtained when a cancer selected from the group consisting of non-small cell lung cancer or colorectal cancer are treated, even more preferably when the cancer is mutated in KRAS, more preferably having a KRAS G12C mutation. In a preferred embodiment, the ratio of component (i):component (ii), more preferably the ratio of Omomyc:adagrasib, ranges from 100:1 to 2,000,000:1 in non-small cell lung cancer, preferably having a KRAS mutation, more preferably having a KRAS G12C mutation, more preferably from 125:1 to 2,000,000:1; more preferably from 140:1 to 1,900,000:1; more preferably from 143:1 to 1,813,190:1; more preferably from 260:1 to 1,900,000:1; more preferably from 268:1 to 1,813,190:1; more preferably from 260:1 to 1,800,000:1; more preferably from 140:1 to 18,000:1; more preferably from 143:1 to 17,679:1; more preferably from 140:1 to 17,000:1; more preferably from 3,500:1 to 925,000:1; more preferably from 3,683:1 to 906,595:1; more preferably from 3,500:1 to 900,000:1. In a preferred embodiment, the ratio of component (i):component (ii), more preferably the ratio of Omomyc:adagrasib, ranges from 1,000:1 to 300,000:1 in colorectal cancer, preferably having a KRAS mutation, more preferably having a KRAS G12C mutation, more preferably from 2,000:1 to 300,000:1; more preferably from 3,000:1 to 250,000:1; more preferably from 3,500:1 to 250,000:1; more preferably from 3,508:1 to 226,649:1; even more preferably from 3,600:1 to 225,000:1.

In a preferred embodiment, the ratio of component (i): component (ii), more preferably the ratio of Omomyc:MRTX1133, ranges from 500:1 to 2,000,000:1; preferably from 800:1 to 2,800,000:1; more preferably from 889:1 to 1,778,133:1; more preferably from 900:1 to 1,700,000:1. Although these ratios are valid for treating any kind of cancer, more preferably, these ratios are obtained when a cancer selected from the group consisting of non-small cell lung cancer or colorectal cancer are treated, even more preferably when the cancer is mutated in KRAS, more preferably having a KRAS G12D mutation. In a preferred embodiment, the ratio of component (i):component (ii), more preferably the ratio of Omomyc:MRTX1133, ranges from 10,000:1 to 500,000:1 in non-small cell lung cancer; preferably having a KRAS mutation, more preferably having a KRAS G12D mutation, more preferably from 12,000:1 to 480,000:1; more preferably from 14,000:1 to 460,000:1; more preferably from 14,340:1 to 455,932:1; even more preferably from 14,500:1 to 450,000:1; even more preferably from 15,000:1 to 440,000:1. In a preferred embodiment, the ratio of component (i):component (ii), more preferably the ratio of Omomyc:MRTX1133, ranges from 500:1 to 2,000,000:1 in colorectal cancer, preferably having a KRAS mutation, more preferably having a KRAS G12D mutation, more preferably from 800:1 to 1,800,000:1; more preferably from 889:1 to 1,778,133:1; even more preferably from 900:1 to 1,700,000:1; even more preferably from 950:1 to 1,600,000:1.

In a preferred embodiment, the ratio of component (i):component (ii), more preferably the ratio of Omomyc:BAY-293, ranges from 10:1 to 10,000:1; preferably from 20:1 to 8,000:1; more preferably from 24:1 to 6,105:1; even more preferably from 25:1 to 6,000:1. Although these ratios are valid for treating any kind of cancer, more preferably, these ratios are obtained when a cancer selected from the group consisting of non-small cell lung cancer or colorectal cancer are treated, even more preferably when the cancer is mutated in KRAS, more preferably having a KRAS G12C mutation or a KRAS G12D mutation. In a preferred embodiment, the ratio of component (i):component (ii), more preferably the ratio of Omomyc:BAY-293, ranges from 20:1 to 10,000:1 in non-small cell lung cancer, preferably having a KRAS mutation, more preferably having a KRAS G12 mutation or a KRAS G12D mutation, more preferably from 24:1 to 6,500:1; more preferably from 24:1 to 6,105:1; more preferably from 25:1 to 6,000:1; even more preferably from 24:1 to 3,500:1; more preferably from 24:1 to 3,052:1; even more preferably from 25:1 to 3,000:1. In a preferred embodiment, the ratio of component (i):component (ii), more preferably the ratio of Omomyc:BAY-293, ranges from 40:1 to 800:1 in colorectal cancer, preferably having a KRAS mutation, more preferably having a KRAS G12C mutation or a KRAS G12D mutation, more preferably from 48:1 to 763:1, even more preferably from 50:1 to 700:1.

In a preferred embodiment, the ratio of component (i):component (ii), more preferably the ratio of Omomyc:BI-3406, ranges from 1:1 to 10,000:1; more preferably from 2:1 to 8,000:1; more preferably from 3:1 to 6,000:1; even more preferably from 3:1 to 5,922:1; even more preferably from 3:1 to 5,500:1. Although these ratios are valid for treating any kind of cancer, more preferably, these ratios are obtained when a cancer selected from the group consisting of non-small cell lung cancer or colorectal cancer are treated, even more preferably when the cancer is mutated in KRAS, more preferably having a KRAS G12C mutation or a KRAS G12D mutation. In a preferred embodiment, the ratio of component (i):component (ii), more preferably the ratio of Omomyc:BI-3406, ranges from 6:1 to 6,000:1 in non-small cell lung cancer, preferably having a KRAS mutation, more preferably having a KRAS G12C mutation or a KRAS G12D mutation, more preferably from 6:1 to 5,922:1; even more preferably from 6:1 to 1,000:1; even more preferably from 6:1 to 500:1; even more preferably from 6:1 to 400:1; even more preferably from 6:1 to 385:1; even more preferably from 6:1 to 350:1.

Preferably these ratios are w/w ratios.

The components of the pharmaceutical composition or the combination of the invention can be administered simultaneously. "Simultaneous administration" encompasses coadministration of the two therapeutic agents, regardless of the relative frequencies or timing of the administration of the respective agents. Thus, simultaneous administration encompasses the coadministration of the two therapeutic agents at the same time and at the same frequencies of administration. In addition, simultaneous administration refers to the coadministration of the two therapeutic agents, in which one agent is administered more frequently than the other(s). In addition, simultaneous administration refers to the coadministration of the two therapeutic agents, in which one agent is administered only once during the administration of the other agent(s).

In an embodiment component (i) is administered intranasally. In another embodiment component (i) is administered intravenously. In another embodiment, component (ii) is administered orally. In another embodiment, component (ii) is administered parenterally, particularly intraperitoneally or intravenously.

In a preferred embodiment component (i) of the combination or pharmaceutical composition of the invention is administered intravenously, whereas the KRAS inhibitor is administered orally. For intravenous administration the preferred dose of component (i) of the combination or composition of the invention, preferably the preferred dose of the polypeptide or functionally equivalent variant thereof, fusion protein or conjugate ranges from 0.01 to 250 mg/Kg which can be administered in single or multiple doses, more preferably between 0.1 to about 100 mg/kg per day. The preferred dose of the KRAS inhibitor for oral administration is 0.01 to 200 mg/Kg, preferably 0.01 to 150 mg/Kg, more preferable between 0.1 to 100 mg/Kg, more preferably 0.5 to 100 mg/Kg, even more preferably 10 to 100 mg/kg, most preferably 50 to 100 mg/Kg. In an embodiment the dose of the KRAS inhibitor for oral administration is 1 mg/kg to 25 mg/kg, more preferably 3 mg/kg to 20 mg/kg, more preferably is 5 mg/kg to 20 mg/kg, more preferably is 8 mg/kg to 18 mg/kg, even more preferably is 10 mg/kg to 18 mg/kg. Preferably said KRAS inhibitor is administered 7 days a week for 4 weeks.

In another embodiment components (i) and (ii) of the combination or pharmaceutical composition of the invention are administered intravenously.

The pharmaceutical composition of the invention can also contain one or several additional compounds for the prevention and/or treatment of pathologies in which there is an uncontrolled cell division, such as cancer. Said additional compounds, such as antitumoral agents can form part of the pharmaceutical composition as independent entities. In a preferred embodiment, the combinations or pharmaceutical compositions of the invention comprise one or more antitumoral agents selected from the group consisting of a cytotoxic agent, an antiangiogenic agent, an antimetastatic agent and an antiproliferative agent.

The pharmaceutical composition of the invention also contain one or several additional pharmaceutically acceptable excipients. "Pharmaceutically acceptable excipient" is understood a therapeutically inactive substance said to be used for incorporating the active ingredient and which is acceptable for the patient from a pharmacological/toxicological point of view and for the pharmaceutical chemist who manufactures it from a physical/chemical point of view with respect to the composition, formulation, stability, acceptation of the patient and bioavailability. The excipient can be a carrier. As used herein "carrier" is meant any substance that serves to improve the delivery and the effectiveness of the active principle within the pharmaceutical composition. In a preferred embodiment, the carrier does not allow direct delivery of component (i) and/or (ii) to the cytoplasm of the cells, i.e. the carrier is not capable of fusing with the plasmatic membrane of the target cells. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the combination or composition. Pharmaceutically acceptable carriers may further comprise minor amounts of auxiliary substances such as wetting or emulsifying agents, preservatives or buffers, which enhance the shelf life or effectiveness of components forming part of the combinations or compositions of the invention. Examples of proper carriers are well known in the literature (see for example Remington's Pharmaceutical Sciences, 19th ed., Mack Publishing Company, Easton, PA, 1995). Examples of carriers without limitation are a series of saccharide such as lactose, dextrose, sucrose, sorbitol, mannitol, xylitol, erythritol, and maltitol; a series of starch such as corn starch, wheat starch, rice starch, and potato starch; a series of cellulose such as cellulose, methyl cellulose, sodium carboxy methyl cellulose, and hydroxyl propylmethyl cellulose; and a series of filler such as gelatin and polyvinyl pyrrolidone. In some cases, a disintegrants such as cross-linked polyvinyl pyrrolidone, agar, alginic acid, or sodium alginate may be added.

The number and the nature of the pharmaceutically acceptable excipients depend on the desired dosage form. The pharmaceutically acceptable excipients are known by the person skilled in the art (Fauli y Trillo C. (1993) "Tratado de Farmacia Galenica", Luzán 5, S.A. Ediciones, Madrid). Said compositions can be prepared by means of the conventional methods known in the state of the art ("Remington: The Science and Practice of Pharmacy", 20th edition (2003) Genaro A.R., ed., Lippincott Williams & Wilkins, Philadelphia, US).

For pharmaceutical compositions comprising an agent that is a nucleic acid molecule, the nucleic acid molecule may be present within any of a variety of delivery systems known to those of ordinary skill in the art, including nucleic acid, and bacterial, viral and mammalian expression systems such as, for example, recombinant expression constructs as provided herein. Techniques for incorporating DNA into such expression systems are well known to those of ordinary skill in the art. The DNA may also be "naked," as described, for example, in Ulmer et al., Science 259:1745-49, 1993 and reviewed by Cohen, Science 259:1691-1692, 1993. The uptake of naked DNA may be increased by coating the DNA onto biodegradable beads, which are efficiently transported into the cells.

Nucleic acid molecules may be delivered into a cell according to any one of several methods described in the art (see, e.g., Akhtar et al., Trends Cell Bio. 2:139 (1992); Delivery Strategies for Antisense Oligonucleotide Therapeutics, ed. Akhtar, 1995, Maurer et al., Mol. Membr. Biol. 16:129-40 (1999); Hofland and Huang, Handb. Exp. Pharmacol. 137:165-92 (1999); Lee et al., ACS Symp. Ser. 752:184-92 (2000); U.S. Pat. No. 6,395,713; International Patent Application Publication No. WO 94/02595); Selbo et al., Int. J. Cancer 87:853-59 (2000); Selbo et al., Tumour Biol. 23:103-12 (2002); U.S. Patent Application Publication Nos. 2001/0007666, and 2003/077829). Such delivery methods known to persons having skill in the art, include, but are not restricted to, encapsulation in liposomes, by iontophoresis, or by incorporation into other vehicles, such as biodegradable polymers; hydrogels; cyclodextrins (see, e.g., Gonzalez et al., Bioconjug. Chem. 10: 1068-74 (1999); Wang et al., International Application Publication Nos. WO 03/47518 and WO 03/46185); poly(lactic-co-glycolic)acid (PLGA) and PLCA microspheres (also useful for delivery of peptides and polypeptides and other substances) (see, e.g., U.S. Pat. No. 6,447,796; U.S. Patent Application Publication No. 2002/130430); biodegradable nanocapsules; and bioadhesive microspheres, or by proteinaceous vectors (International Application Publication No. WO 00/53722). In another embodiment, the nucleic acid molecules can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives (see also, e.g., U.S. Patent Application Publication No. 2003/0077829).

In a particular embodiment, when the composition or combination according to the invention comprises a nucleic acid (DNA, RNA, siRNA, antisense oligonucleotides, ribozimes, aptamers and spiegelmers), the pharmaceutical composition may be formulated as a composition intended for use in gene therapy; by way of illustration, not limitation, that pharmaceutical composition may contain a viral or nonviral vector, which comprises the suitable polynucleotide or gene construction. By way of illustration and not limitation, said vectors, may be viral, for example, based on retrovirus, adenovirus, etc., or nonviral such as ADN-liposome, ADN-polymer, ADN-polymer-liposome complexes, etc. [see "Nonviral Vectors for Gene Therapy", edited by Huang, Hung and Wagner, Academic Press (1999)]. Said vectors, which contain the corresponding polynucleotide or gene construction, may be administered directly to a subject by conventional methods. Alternatively, said vectors may be used to transform, or transfect or infect cells, for example, mammal cells, including human, *ex vivo,* which subsequently will be implanted into a human body or an animal to obtain the desired therapeutic effect. For administration to a human body or an animal, said cells will be formulated in a suitable medium that will have no adverse influence on cell viability.

The combination or pharmaceutical composition of the invention can be administered by any type of suitable route, such as by oral route, topical route, by inhalation or parenteral route so that the pharmaceutically acceptable excipients necessary for the formulation of the desired dosage form will be included. Other routes of administration can be rectally, intracisternally or intravaginally. The preferred route of administration of said combination or pharmaceutical compositions is the endovenous route for component (i) and the oral route for component (ii). Alternatively, the route of administration of said combination or pharmaceutical composition is the intravenous route.

"Oral route" is understood as the pharmaceutical composition incorporated into the organism after deglutition. In a particular embodiment, the pharmaceutical composition of the invention can be in a dosage form suitable for its administration by oral route, whether it is solid or liquid. The dosage forms suitable for their administration by oral route can be tablets, capsules, syrups or solutions, and can contain any conventional excipient known in the art, such as binders, for example syrup, acacia, gelatin, sorbitol or polyvinylpyrrolidone; filling agents, for example lactose, sugar, corn starch, calcium phosphate, sorbitol or glycine; lubricants for compression, for example, magnesium stearate; disintegrating agents, for example starch, polyvinylpyrrolidone, sodium glycolate of starch or microcrystalline cellulose; or pharmaceutically acceptable wetting agents such as sodium lauryl sulfate. The solid oral compositions can be prepared by means of conventional processes of mixing, filling or compressing. Repetitive mixing operations can be used to completely distribute the active agent in those compositions that use high amounts of filling agents. Said operations are conventional in the art. The tablets can be prepared, for example, by means of wet or dry granulation, and optionally coating them according to the processes known in the common pharmaceutical practice, particularly with an enteric coating.

On the other hand, "topical route" is understood as an administration by non-systemic route, and includes the application of a pharmaceutical composition of the invention externally on the epidermis, in the oral cavity and the instillation of said composition into ears, eyes and nose, and in which it does not significantly enter the blood stream. Dosage forms for topical or transdermal administration of a compound of this invention include ointments, pastes, creams, lotions, gels, powders, solutions, sprays, inhalants or patches.

Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

In an embodiment, the combination or pharmaceutical composition is administered systemically.

"Systemic route" is understood as the administration by oral route, intravenous route, intraperitoneal route and intramuscular route. The amount of components (i) and (ii) required for the therapeutic or prophylactic effect will naturally vary according to the elected compound, the nature and the severity of the illness that is going to be treated, and the patient. Preferably the combination or pharmaceutical composition is administered orally.

In another embodiment, the combination or pharmaceutical composition is administered intranasally. In a preferred embodiment, the intranasal administration is performed by instillation or nasal inhalation.

"Inhalation" is understood as the administration by intranasal route and by oral inhalation. The dosage forms suitable for said administration, such as a formulation in aerosol or a meter dosed inhaler can be prepared by means of conventional techniques. In an embodiment the route of administration is the intranasal route.

As it is used herein, the term "parenteral", includes administration by intravenous route, intraperitoneal route, intramuscular route or subcutaneous route. Subcutaneous, intramuscular and intravenous dosage forms of parenteral administration are generally preferred. In an embodiment, the combination or pharmaceutical composition is administered intravenously.

In one embodiment, the combinations or pharmaceutical compositions of the invention can be adapted for their parenteral administration, such as sterile solutions, suspensions or lyophilized products in the appropriate dosage unit form. The combinations or pharmaceutical compositions suitable for its injectable use include sterile aqueous solutions (when they are soluble in water), or dispersions and sterile powders for the extemporaneous preparation of sterile injectable solutions or dispersions. For its administration by intravenous route, some suitable carriers include saline solution buffered with phosphate (PBS). In all the cases, the combination or composition must be sterile, and must be fluid to the point which that there exists easy ability for being injected. It must be stable in the preparation and storage conditions, and must be protected from the contamination action of microorganisms such as bacteria and fungi. The carrier can be a solvent or a dispersion medium which contains, for example, water, ethanol, a pharmaceutically acceptable polyol such as glycerol, propylene glycol, liquid polyethylene glycol and suitable mixtures thereof. Suitable fluidity can be maintained, for example, by means of using a coating such as lecithin, by means of maintaining the particle size required in the case of dispersion and by means of using surfactants. The prevention of the action of the microorganisms can be achieved by means of various antibacterial and antifungal agents, for example, parabens, chlorobutanol, phenol, ascorbic acid, thiomersal, and the like. In most cases, it will be preferable to include isotonic agents, for example, sugars; polyalcohols such as mannitol, sorbitol; or sodium chloride in the composition. The prolonged absorption of the injectable compositions may be caused by the inclusion of an agent which delays the absorption, for example, aluminum and gelatin monostearate.

The injectable sterile solutions can be prepared by incorporating the active compound in the required amount in a suitable solvent with one or a combination of the aforementioned ingredients, as needed, followed by sterilization by filtration through sterile membranes. Generally, the dispersions are prepared by incorporating the active compound in a sterile vehicle containing a basic dispersion medium and the rest of the ingredients required from among those previously listed. In the case of sterile powders for the preparation of injectable sterile solutions, the preferred preparation processes are vacuum drying and lyophilization which give rise to a powder with the active ingredient plus any desired additional ingredient from a previously filtered sterile solution thereof.

The combinations or pharmaceutical compositions of the invention can be suitably administered by means of pulse infusion, for example, with decreasing doses of the composition. Preferably, the dose is administered by means of injections, more preferably intravenous or subcutaneous injections, partly depending if the administration is acute or chronic.

Alternatively, as mentioned above, the different components of the composition are differently administered.

Thus, in an embodiment component (i) of the combination or composition, preferably the polypeptide or functionally equivalent variant or the conjugate of the invention, is administered intravenously, while the KRAS inhibitor is administered orally.

In another embodiment both components (i) and (ii) are administered intravenously.

In another embodiment, component (i) of the combination or composition, preferably the polypeptide or functionally equivalent variant thereof, or the conjugate of the composition, is administered intranasally or by inhalation.

Dosage forms of compositions intended for intranasal and intrapulmonary administration are preferably a liquid, a suspension or a solid. A suspension is a liquid preparation containing solid particles dispersed in a liquid vehicle. The dosage forms are preferably metered. For example, metered drops/sprays mean that the dispenser that includes the drops/spray delivers the drops/spray containing a metered dose (a predetermined quantity) of the composition for use according to the invention.

One preferred dosage form in the context of the intranasal administration route includes nasal drops. Drops are deposited mostly in the posterior portion of the nose and thus removed rapidly into the nasal pharynx. A concern with drops is often how to precisely control the drug's dose which is particularly important for the administration of the composition.

Another intranasal dosage form by which the pharmaceutical composition of the invention can be administered is nasal sprays. Nasal sprays typically contain the conjugate dissolved or suspended in a solution or a mixture of excipients (e.g. preservatives, viscosity modifiers, emulsifiers, buffering agents) in a non-pressurized dispenser. Nasal sprays have several advantages including compactness of the delivery device, convenience, simplicity of use, and accuracy of delivering dosages of 25 to 200 pL. They are deposited in the anterior portion of the nose and cleared slowly into nasal pharynx by mucociliary clearance. The nasal spray as used herein can be a liquid or a suspension.

Another intranasal dosage form is a nasal aerosol. Nasal aerosols differ from nasal sprays by the method of the composition dispensing: in aerosols, a compound is dispensed due to an excess of pressure and releases through a valve. In sprays, a compound is dispensed due to forcing away by a micropump bucket, while the pressure in the vial is similar to atmosphere pressure. Aerosols have similar advantages as sprays.

The composition according to the invention may alternatively preferably be administered by nasal emulsions, ointments, gels, pastes or creams. These are highly viscous solutions or suspensions applied to the nasal mucosa.

Due to the limited volume of the composition that can be efficiently delivered to the nasal mucosa, liquid intranasal dosage forms usually have higher concentrations as the corresponding intravenous dosage forms. When substances become poorly soluble or are instable in liquid form, powders can be used to administer the composition of the invention. Further advantages of powders are that they do not require preservatives and have usually a higher stability as compared to liquid formulations. The main limitation on intranasal powder application is related to its irritating effect on the nasal mucosa.

One dosage form in context of intrapulmonary administration is an inhalation aerosol. Inhalation aerosols are usually packaged under pressure and contain the composition according to the invention which is released upon activation of a valve system into the respiratory tract, in particular the lungs. The released aerosol is a colloid of fine solid particles (suspension) or liquid droplets (solution) in air or another gas. Accordingly, the aerosol may be a solution or a suspension aerosol. The liquid droplets or solid particles have preferably a diameter of less than 100 pm, more preferably less than 10 pm, most preferably less than 1 pm.

Another dosage form in context of intrapulmonary administration is inhalation sprays. Inhalation sprays are typically aqueous based and do not contain any propellant. They deliver the conjugate to the lungs by oral inhalation.

Nebulized inhalation solutions and suspensions may also be used to deliver the conjugate by the intrapulmonary route. Nebulized inhalation solutions and suspensions are typically aqueous-based formulations that contain the composition according to the invention. The nebulized inhalation solutions and suspensions deliver the composition to the lungs by oral inhalation for systemic effects and are used with a nebulizer.

Dry powder inhalation is an alternative to aerosol inhalation. The composition is usually included in a capsule for manual loading or within the inhaler. Dry powders are typically delivered by an inhaler to the lungs by oral inhalation. The dry powders as used herein can be formulated neat. Neat formulations contain the drug alone or quasi-alone e.g. as spry dried powder. The dry powders as used herein can be also formulated with a carrier such as lactose.

Intrapulmonary dosage forms are preferably metered, i.e. are delivered to the lungs in a predetermined quantity.

Devices for intranasal delivery in the context of the present invention include spray pump systems, pipettes for delivering drops, metered-dose spray pumps, nasal pressurized metered-dose inhalers, powder spray systems, breath-actuated powder inhalers and nasal powder insufflators. The intranasal delivery device may be filled with a single dose amount or a multi-dose amount of the intranasal formulation.

Using the intrapulmonary route the conjugate may be administered with a metered dose inhaler. A metered-dose inhaler (MDI) provides a fine mist of conjugate, generally with an aerodynamic particle size of less than 5 pm.

Dry powder inhalers can be alternatively used to deliver the composition intrapulmonary. Dry powder inhalers present powders as single-dose or multidose powders.

Another device for intrapulmonary delivery is a nebulizer including ultrasonic and air jet nebulizers. In ultrasonic nebulizers, ultrasound waves are formed in an ultrasonic nebulizer chamber by a ceramic piezoelectric crystal that vibrates when electrically excited. This generates an aerosol cloud at the solution surface. The aerosol produced by an air jet nebulizer is generated when compressed air is forced through an orifice. A liquid may be withdrawn from a perpendicular nozzle (the Bernoulli Effect) to mix with the air jet which is atomized using baffles to facilitate the formation of the aerosol cloud.

In one embodiment, each of the components of the combination or the pharmaceutical composition of the invention is prepared with carriers which will protect the components, particulary component (i), from a rapid elimination from the body, such as a controlled release formulation, including implants and microencapsulated administration systems. Biodegradable biocompatible polymers such as ethylene vinylacetate, polyanhydrides, polyglycolic acid, collagen, polyorthoesters and polylactic acid can be used. The processes for preparing said formulations will be clear for persons skilled in the art. The materials can also be commercially obtained in Alza Corporation and Nova Pharmaceuticals, Inc.

The sustained release compositions also include preparations of crystals suspended in suitable formulations which can maintain the crystals in suspension. These preparations, when they are injected by subcutaneous or intraperitoneal route may produce a sustained release effect. Other compositions also include the components (i) and/or (ii) trapped in liposomes. The liposomes containing such components are prepared by means of known methods such as Epstein et al., Proc. Natl. Acad. Sci. USA, (1985) 82:3688-3692; Hwang et al., Proc. Natl. Acad. Sci. USA, (1980) 77:4030-4034; EP 52,322; EP 36,676; EP 88,046; EP 143,949. In a preferred embodiment, components (i) and/or (ii) are contained in liposomes, preferably both components are contained in liposomes, more preferably in the same liposome.

Despite the fact that Omomyc, functionally equivalent variants thereof, conjugates and fusion proteins of the invention are capable of translocating across biological membranes, it is possible to formulate Omomyc, any of its functionally equivalent variants, conjugates, polynucleotides, vectors or cells in nanoparticles. The nanoparticles may contribute to preserve the integrity of the components in the biological fluids until it reaches the target organ. Moreover, in the case of compositions comprising component (ii) or other antitumor agent, encapsulation of the composition may decrease secondary effects caused by the antitumor agent. In addition, nanoparticles can also be modified so as to include moieties which allow the targeting of the nanoparticle to an organ of interest. In this way, component (i) of the combination or the composition of the invention will be delivered in the proximity of the target organ, facilitating access of component (i) to the interior of the cells where its biological activity is required.

Thus, in another embodiment, component (i) of the combination or composition of the invention is provided forming part of a nanoparticle. In another embodiment, both components of the combination or composition of the invention are provided forming part of a nanoparticle, preferably both components are provided inside the same nanoparticle.

As used herein, the term "nanoparticle" refers to any material having dimensions in the 1-1,000 nm range. In some embodiments, nanoparticles have dimensions in the 2-200 nm range, preferably in the 2-150 nm range, and even more preferably in the 2-100 nm range. Nanoparticles that can be used in the present invention include such nanoscale materials as a lipid-based nanoparticle, a superparamagnetic nanoparticle, a nanoshell, a semiconductor nanocrystal, a quantum dot, a polymer-based nanoparticle, a silicon-based nanoparticle, a silica-based nanoparticle, a metal-based nanoparticle, a fullerene and a nanotube. Molecules can be either embedded in the nanoparticle matrix or may be adsorbed onto its surface, preferably molecules are embedded in the nanoparticle.

In a preferred embodiment, the nanoparticle is a liposome.

Targeted delivery can be achieved by the addition of ligands without compromising the ability of nanoparticles to deliver their content. It is contemplated that this will enable delivery to specific cells, tissues and organs. The targeting specificity of the ligand-based delivery systems are based on the distribution of the ligand receptors on different cell types. The targeting ligand may either be non-covalently or covalently associated with a nanoparticle, and can be conjugated to the nanoparticles by a variety of methods as discussed herein.

Examples of proteins or peptides that can be used to target nanoparticles include transferin, lactoferrin, TGF-β, nerve growth factor, albumin, HIV Tat peptide, RGD peptide, and insulin, as well as others.

It will be understood that the formulations of the invention in a nanoparticle are not intended or are not solely intended for facilitating the access of the components (i) and/or (ii) to the interior of the cell but to protect components (i) and/or (ii) from degradation and/or for facilitating targeting of the nanoparticle to the organ of interest.

In one example, the nanoparticle may be made up of a biodegradable polymer such as poly(butylcyanoacrylate) (PBCA). Examples of elemental nanoparticles include carbon nanoparticles and iron oxide nanoparticles, which can then be coated with oleic acid (OA)-Pluronic(R). In this approach, a drug (e.g., a hydrophobic or water insoluble drug) is loaded into the nanoparticle. Other nanoparticles are made of silica.

Nanoparticles can be formed from any useful polymer. Examples of polymers include biodegradable polymers, such as poly(butyl cyanoacrylate), poly(lactide), poly(glycolide), poly-s-caprolactone, poly(butylene succinate), poly(ethylene succinate), and poly(p-dioxanone); poly(ethyleneglycol); poly-2-hydroxyethylmethacrylate (poly(HEMA)); copolymers, such as poly(lactide-co-glycolide), poly(lactide)-poly(ethyleneglycol), poly(poly(ethyleneglycol)cyanoacrylate-cohexadecylcyanoacrylate, and poly [HEMA-co-methacrylic acid]; proteins, such as fibrinogen, collagen, gelatin, and elastin; and polysaccharides, such as amylopectin, a amylose, and chitosan.

Other nanoparticles include solid lipid nanoparticles (SLN). Examples of lipid molecules for solid lipid nanoparticles include stearic acid and modified stearic acid, such as stearic acid-PEG 2000; soybean lecithin; and emulsifying wax. Solid lipid nanoparticles can optionally include other components, including surfactants, such as Epicuron(R) 200, poloxamer 188 (Pluronic(R) F68), Brij 72, Brij 78, polysorbate 80 (Tween 80); and salts, such as taurocholate sodium. Agents can be introduced into solid lipid nanoparticles by a number of methods discussed for liposomes, where such methods can further include high-pressure homogenization, and dispersion of microemulsions.

Nanoparticles can also include nanometer-sized micelles. Micelles can be formed from any polymers described herein. Exemplary polymers for forming micelles include block copolymers, such as poly(ethylene glycol) and poly(ε-caprolactone). (e.g.,a PEO- b-PCL block copolymer including a polymer of ε-caprolactone and α-methoxy-ω-hydroxy-poly(ethylene glycol)).

In certain embodiments, the properties of the nanoparticles are altered by coating with a surfactant. Any biocompatible surfactant may be used, for example, polysorbate surfactants, such as polysorbate 20, 40, 60, and 80 (Tween 80); Epicuron(R) 200; poloxamer surfactants, such as 188 (Pluronic(R) F68) poloxamer 908 and 1508; and Brij surfactants, such as Brij 72 and Brij 78.

Nanoparticles can optionally be modified to include hydrophilic polymer groups (e.g., poly(ethyleneglycol) or poly(propyleneglycol)), for example, by covalently attaching hydrophilic polymer groups to the surface or by using polymers that contain such hydrophilic polymer groups (e.g., poly[methoxy poly (ethyleneglycol) cyanoacrylate-co-hexadecyl cyanoacrylate]). Nanoparticles can be optionally cross linked, which can be particularly useful for protein-based nanoparticles.

In another embodiment, the pharmaceutical composition of the invention is a nanoemulsion. "Nanoemulsion" as used herein means a colloidal dispersion of droplets (or particles) which at least some of the droplets have diameters in the nanometer size range. The nanoemulsions are comprised of omega-3, -6 or -9 fatty acid rich oils in an aqueous phase and thermo-dynamically stabilized by amphiphilic surfactants, which make up the interfacial surface membrane, produced using a high shear microfluidization process usually with droplet diameter within the range of about 80-220 nm.

### Therapeutic uses of the invention

In an aspect, the invention relates to the combination or the pharmaceutical composition of the invention for use in medicine.

In a further aspect, the invention relates to the combination or the pharmaceutical composition of the invention for use in the prevention and/or treatment of cancer.

In another aspect, the invention refers to the combination or the pharmaceutical composition of the invention for the preparation of a medicament for the prevention and/or treatment of cancer.

In another aspect, the invention also refers to a method for the prevention and/or treatment of cancer that comprises administering to a subject in need thereof a therapeutically effective amount of the combination or pharmaceutical composition of the invention.

In a preferred embodiment, the preventive or therapeutic method according to the invention involves the direct use of a combination or composition comprising a polypeptide comprising Omomyc, a functionally equivalent variant thereof, a conjugate or a fusion protein. Thus, in a preferred embodiment, the preventive or therapeutic methods according to the invention do not involve the administration of the nucleic acid encoding a polypeptide comprising Omomyc or the functionally equivalent variant thereof or fusion protein, or the administration of a vector encoding this nucleic acid, or a cell comprising said nucleic acid.

"Prevention" is understood as the administration of a combination or composition of the invention in an initial or early stage of the disease, or to also prevent its onset.

The term "treatment" is used to designate the administration of a combination or composition of the invention to control the progression of the disease before or after the clinical signs have appeared. Control of the progression of the disease is understood as the beneficial or desired clinical results which include but are not limited to reduction of the symptoms, reduction of the duration of the disease, stabilization of pathological conditions (specifically avoiding additional impairment), delaying the progression of the disease, improving the pathological condition and remission (both partial and complete). The control of the progression of the disease also involves a prolongation of survival in comparison to the expected survival if the treatment was not applied. In a preferred embodiment, control of the progression of the disease is measured as healthy lung/thorax volume ratio. In another embodiment, control of the progression of the disease is measured as reduction in tumor volume. In another embodiment, control of the progression of the disease is measured as reduction in tumor cell viability.

The term "cancer" is referred to a disease characterized by uncontrolled cell division (or by an increase of survival or apoptosis resistance), by the ability of said cells to invade other neighbouring tissues (invasion) or by the spread to other areas of the body where the cells are not normally located (metastasis) through the lymphatic and blood vessels. Depending on whether or not tumours can spread by invasion and metastasis, they are classified as being either benign or malignant: benign tumours are tumours that cannot spread by invasion or metastasis, i.e., they only grow locally; whereas malignant tumours are tumours that are capable of spreading by invasion and metastasis. The methods according to the present invention are useful for the treatment of local and malignant tumours.

Cancer includes, in one embodiment, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), hairy cell leukemia, polycythemia vera, lymphoma (e.g., Hodgkin's disease or non-Hodgkin's disease), AIDS-associated leukemias, Waldenstrom's macroglobulinemia, multiple myeloma, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, mendotheliosarcoma, lymphangiosarcoma, lymphangioendotheliosarcoma, synovioma, mesothelioma, Ewing's tumor, leiomyosarcoma, rhabdomyosarcoma, Kaposi's sarcoma, colon carcinoma, pancreatic cancer, breast cancer, biliary tract cancer, esophageal cancer, ovarian cancer, prostate cancer, oral cancer including squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, teratoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, intraepithelial neoplasms including Bowen's disease and Paget's disease, neuroglioma, glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, and retinoblastoma).

In some embodiments, the cancer is glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, or retinoblastoma.

In some embodiments, the cancer is acoustic neuroma, astrocytoma (e.g. Grade I - Pilocytic Astrocytoma, Grade II - Low-grade Astrocytoma, Grade III - Anaplastic Astrocytoma, or Grade IV - Glioblastoma (GBM)), chordoma, CNS lymphoma, craniopharyngioma, brain stem glioma, ependymoma, mixed glioma, optic nerve glioma, subependymoma, medulloblastoma, meningioma, metastatic brain tumor, oligodendroglioma, pituitary tumors, primitive neuroectodermal (PNET) tumor, or schwannoma. In some embodiments, the cancer is a type found more commonly in children than adults, such as brain stem glioma, craniopharyngioma, ependymoma, juvenile pilocytic astrocytoma (JPA), medulloblastoma, optic nerve glioma, pineal tumor, primitive neuroectodermal tumors (PNET), or rhabdoid tumor. In some embodiments, the patient is an adult human. In some embodiments, the patient is a child or pediatric patient.

Cancer includes, in another embodiment, without limitation, mesothelioma, hepatobilliary (hepatic and billiary duct), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, non-Hodgkins's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

In some embodiments, the cancer is selected from hepatocellular carcinoma, ovarian cancer, ovarian epithelial cancer, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical adenoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

In some embodiments, the cancer is selected from hepatocellular carcinoma (HCC), hepatoblastoma, colon cancer, rectal cancer, ovarian cancer, ovarian epithelial cancer, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical adenoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In some embodiments, a cancer is a solid tumor, such as a sarcoma, carcinoma, or lymphoma. Solid tumors generally comprise an abnormal mass of tissue that typically does not include cysts or liquid areas. In some embodiments, the cancer is selected from renal cell carcinoma, or kidney cancer; hepatocellular carcinoma (HCC) or hepatoblastoma, or liver cancer; melanoma; breast cancer; colorectal carcinoma, or colorectal cancer; colon cancer; rectal cancer; anal cancer; lung cancer, such as non-small cell lung cancer (NSCLC) or small cell lung cancer (SCLC); ovarian cancer, ovarian epithelial cancer, ovarian carcinoma, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical carcinoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

In some embodiments, the cancer is selected from hepatocellular carcinoma (HCC), hepatoblastoma, colon cancer, rectal cancer, ovarian cancer, ovarian epithelial cancer, ovarian carcinoma, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical carcinoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In some embodiments, the cancer is hepatocellular carcinoma (HCC). In some embodiments, the cancer is hepatoblastoma. In some embodiments, the cancer is colon cancer. In some embodiments, the cancer is rectal cancer. In some embodiments, the cancer is ovarian cancer, or ovarian carcinoma. In some embodiments, the cancer is ovarian epithelial cancer. In some embodiments, the cancer is fallopian tube cancer. In some embodiments, the cancer is papillary serous cystadenocarcinoma. In some embodiments, the cancer is uterine papillary serous carcinoma (UPSC). In some embodiments, the cancer is hepatocholangiocarcinoma. In some embodiments, the cancer is soft tissue and bone synovial sarcoma. In some embodiments, the cancer is rhabdomyosarcoma. In some embodiments, the cancer is osteosarcoma. In some embodiments, the cancer is anaplastic thyroid cancer. In some embodiments, the cancer is adrenocortical carcinoma. In some embodiments, the cancer is pancreatic cancer, or pancreatic ductal carcinoma. In some embodiments, the cancer is pancreatic adenocarcinoma. In some embodiments, the cancer is glioma. In some embodiments, the cancer is malignant peripheral nerve sheath tumors (MPNST). In some embodiments, the cancer is neurofibromatosis-1 associated MPNST. In some embodiments, the cancer is Waldenstrom's macroglobulinemia. In some embodiments, the cancer is medulloblastoma.

In some embodiments, a cancer is a viral-associated cancer, including human immunodeficiency virus (HIV) associated solid tumors, human papilloma virus (HPV)-16 positive incurable solid tumors, and adult T-cell leukemia, which is caused by human T-cell leukemia virus type I (HTLV-I) and is a highly aggressive form of CD4+ T-cell leukemia characterized by clonal integration of HTLV-I in leukemic cells (See https://clinicaltrials.gov/ct2/show/study/ NCT02631746); as well as virus-associated tumors in gastric cancer, nasopharyngeal carcinoma, cervical cancer, vaginal cancer, vulvar cancer, squamous cell carcinoma of the head and neck, and Merkel cell carcinoma. (See https://clinicaltrials.gov/ct2/show/study/NCT02488759; see also https://clinicaltrials.gov/ct2/show/study/NCT0240886; https://clinicaltrials.gov/ct2/show/ NCT02426892).

Other cancers will be known to one of ordinary skill in the art.

In a preferred embodiment, the cancer is selected from the group consisting of breast cancer, ovarian cancer, pancreatic cancer, prostate cancer, lung cancer, colorectal cancer, stomach/gastric cancer, endometrial/uterine/cervical cancer, bladder cancer, head and neck cancer, leukemia, sarcoma, cholangiocarcinoma, glioblastoma, multiple myeloma, lymphoma. More preferable, the cancer is selected from the list consisting of breast cancer, ovarian cancer, and prostate cancer, even preferably it is a breast cancer or an ovarian cancer, yet more preferably it is a breast cancer.

In a preferred embodiment, the cancer is a KRAS mutant cancer, i.e., a cancer having a KRAS mutation. More preferably, is a mutant cancer selected from the group consisting of a KRAS G12C mutant cancer, a KRAS G12D mutant cancer and a KRAS G13C mutant cancer.

In another embodiment, the mutation in KRAS is selected from the group consisting of the G12C mutation, the G12D mutation, the G13C mutation, the G12S mutation, the G12V mutation, the G12R mutation, the G12F mutation, the G12I mutation, the G13D mutation, the G13C mutation, the G13R mutation, and the Q61L mutation.

In another preferred embodiment, the cancer is pancreatic cancer, particularly is pancreatic ductal adenocarcinoma.

In another embodiment, the cancer is glioblastoma.

"Glioblastoma", also known as glioblastoma and grade IV astrocytoma, is the most common and most aggressive cancer that begins within the brain.

In another embodiment, the cancer is lung cancer.

The terms "lung cancer" or "lung tumour" refer to the physiological condition in mammals characterized by unregulated cell growth in tissues of the lung. The term lung cancer is meant to refer to any cancer of the lung and includes non-small cell lung carcinomas and small cell lung carcinomas. In an embodiment, the lung cancer is non- small cell lung cancer (NSCLC). In another embodiment, the lung cancer is small cell lung cancer (SCLC).

The term non-small cell lung cancer (NSCLC), as used herein, refers to a group of heterogeneous diseases grouped together because their prognosis and management is roughly identical and includes, according to the histologic classification of the World Health Organization/International Association for the Study of Lung Cancer (Travis WD et al. Histological typing of lung and pleural tumours. 3rd ed. Berlin: Springer-Verlag, 1999):
(i) squamous cell carcinoma (SCC), accounting for 30% to 40% of NSCLC, starts in the larger breathing tubes but grows slower meaning that the size of these tumours varies on diagnosis.
(ii) adenocarcinoma is the most common subtype of NSCLC, accounting for 50% to 60% of NSCLC, which starts near the gas-exchanging surface of the lung and which includes a subtype, the bronchioalveolar carcinoma, which may have different responses to treatment.
(iii) large cell carcinoma is a fast-growing form that grows near the surface of the lung. It is primarily a diagnosis of exclusion, and when more investigation is done, it is usually reclassified to squamous cell carcinoma or adenocarcinoma.
(iv) adenosquamous carcinoma is a type of cancer that contains two types of cells: squamous cells (thin, flat cells that line certain organs) and gland-like cells.
(v) carcinomas with pleomorphic, sarcomatoid or sarcomatous elements. This is a group of rare tumours reflecting a continuum in histologic heterogeneity as well as epithelial and mesenchymal differentiation.
(vi) carcinoid tumour is a slow-growing neuroendocrine lung tumour and begins in cells that are capable of releasing a hormone in response to a stimulus provided by the nervous system.
(vii) carcinomas of salivary gland type begin in salivary gland cells located inside the large airways of the lung.
(viii) unclassified carcinomas include cancers that do not fit into any of the aforementioned lung cancer categories.

In a particular embodiment, the NSCLC is selected from squamous cell carcinoma of the lung, large cell carcinoma of the lung and adenocarcinoma of the lung.

In an even more preferred embodiment, the lung cancer is adenocarcinoma, more preferably a KRas-driven lung adenocarcinoma, preferably a KRAS-mutant adenocarcinoma. In the case of NSCLC, KRAS mutations occur predominantly (95%) at codons 12 (>80%) and 13. The most frequent codon variant, accounting for approximately 39% of KRAS-mutant NSCLC, is the KRAS G12C mutation. Other common mutations include KRAS G12V (18-21%) and KRAS G12D (17-18%) variants. Remarkably, smokers and never smokers have a different spectrum of mutations and codon variants in KRAS. Thus, transition mutations (G > A) are more frequent in never smokers, whereas transversion mutations (G >C or G >T) are more common in former or current smokers. In a preferred embodiment, the KRAS-mutant adenocarcinoma is KRAS G12D mutant. In another preferred embodiment, the KRAS-mutant adenocarcinoma is KRAS G12C mutant.

In one embodiment, the mutation in the KRAS gene is a mutation at the glycine at position 12, at the glycine at position 13 or at the glutamine at position 61. In a more preferred embodiment, the mutation is selected from the group consisting of the G12S mutation, the G12V mutation, the G12D mutation, the G13D mutation, the G12C mutation, the G12R mutation, the G12F mutation, the G12I mutation, the G13C mutation, the G13R mutation, or the Q61L mutation. In a preferred embodiment, the mutation is the G12D mutation. In another embodiment, the lung cancer is a KRas^{GD12}/p53-driven lung cancer, preferably a KRas^{GD12}/p53-driven NSCLC.

The term small cell lung cancer (SCLC), as used herein, refers to a proliferation of small cells with unique and strict morphological features, containing dense neurosecretory granules which give this tumor an endocrine/paraneoplastic syndrome associated. Most cases arise in the larger airways (primary and secondary bronchi). These cancers grow quickly and spread early in the course of the disease.

In another embodiment the cancer is a colorectal cancer.

In another preferred embodiment, the cancer to be treated according to the invention is characterized by expressing increased levels of one or more KRAS proteins, particularly of mutant KRAS proteins. KRAS levels are considered to be increased with respect to a reference value when the levels of KRAS in a sample of said cancer show an increase of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more. The reference value can be the value corresponding to the level of expression of KRAS, particularly mutant KRAS in a non-cancer sample.

In an embodiment, the cancer is a primary tumor. The term "primary tumor", as used herein, refers to a tumor that originated in the location or organ in which it is present and did not metastasize to that location from another location.

In another embodiment, the cancer is a cancer metastasis. In the context of the present invention, "metastasis" is understood as the propagation of a cancer from the organ where it started to a different organ. It generally occurs through the blood or lymphatic system. When the cancer cells spread and form a new tumor, the latter is called a secondary or metastatic tumor. The cancer cells forming the secondary tumor are like those of the original tumor. If a breast cancer, for example, spreads (metastasizes) to the lung, the secondary tumor is formed of malignant breast cancer cells. The disease in the lung is metastatic breast cancer and not lung cancer. The authors of the present invention have also observed that the combination or composition of the invention is capable of decreasing cell proliferation irrespective of whether the cancer shows increased expression or activity of the Myc protein. In a preferred embodiment, the cancer to be prevented or treated is Myc-induced cancer.

In an embodiment, the cancer is a solid tumour.

In another embodiment, the cancer is a cancer resistant to KRAS inhibitors.

"Resistance" is the reduction in effectiveness of a medication in treating a disease or condition. The expression "cancer resistant", a used herein, refers to a cancer having resistance to KRAS inhibitors either innate or acquired resistance. Innate resistance occurs when KRAS inhibitors are ineffective from the start of treatment, due to pre-existing resistance mechanisms. Acquired resistance occurs when KRAS inhibitors become ineffective during the course of treatment and after a clinical benefit has been observed.

All combinations of compounds of the invention and types of cancer are included in the present invention.

In some embodiments, the combination or composition of the invention produces an arresting of the growth of the tumor. In some embodiments, the combination or composition of the invention produces a reduction in the tumor size (e.g., volume or mass) by at least 5%, 10%, 25%, 50%, 75%, 90% or 99% relative to the size of the tumor prior to treatment. In some embodiments, the combination or composition of the invention produces the reduction of the quantity of the tumor in the patient by at least 5%, 10%, 25%, 50%, 75%, 90% or 99% relative to the quantity of the tumor prior to treatment.

A "subject", as used herein, includes any animal that has a cancer or exhibits a symptom of cancer, or is at risk for having a cancer or exhibiting a symptom of cancer. Suitable subjects (patients) include laboratory animals (such as mouse, rat, rabbit, or guinea pig), farm animals, and domestic animals or pets (such as cats or dogs). Non-human primates and, preferably, human patients, are included. Preferably, the subject is a mammal, most preferably a human.

The combinations or compositions for use in the prevention and/or treatment of cancer, may be administered using any amount and any route of administration effective for treating or lessening the severity of a cancer. The exact amount required will vary from subject to subject, depending on the species, age, and general condition of the subject, the severity of the disease or condition, the particular agent, its mode of administration, and the like. Compounds of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compounds and compositions of the present invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts.

In a preferred embodiment, component (i) of the invention, preferably the polypeptide or the functionally equivalent variant thereof, or the conjugate, synergistically interact with the KRAS inhibitor of the combination or composition in treating cancer (to achieve the therapeutic effect).

Particularly, in a more preferred embodiment, the combination or pharmaceutical composition for use in the prevention and/or treatment of cancer is a combination or pharmaceutical composition wherein the polypeptide or the functionally equivalent variant thereof, or the conjugate, is in an amount that synergistically interact with the KRAS inhibitor in treating cancer.

The terms "synergistic effect" or "synergistically interact" are used interchangeably. A synergistic effect is one that is greater than the additive effect that would be predicted by summing the actual effects of the individual agents *in vitro. In vivo,* a synergistic effect is a physiological effect, and particularly a therapeutic effect, that is greater than the additive effect that would be predicted by summing the actual effects of the individual agents *in vivo.*

Thus, if two agents are administered, they together provide a measurable physiological effect, and particularly a therapeutic effect, if the actual effect of the agents together is greater than would be predicted by summing the actual therapeutic effects of the individual agents. Particularly, a synergistic effect is provided when a first agent alone provides some measurable effect, a second agent alone provides some measurable effect, and together the two agents provide a measurable effect greater than the effect provided by the sum of both individual agents. More particularly, a synergistic effect is provided when a first agent alone provides no measurable effect, a second agent alone provides some measurable effect, and together the two agents provide a measurable effect greater than the effect provided by the second agent alone. Still more particularly, a synergistic effect is provided when neither a first agent alone nor a second agent alone provide any measurable effect, but together the two agents provide a measurable effect. Since components (i) and (ii) act synergistically, the amount of components (i) and/or (ii) of the combinations or compositions of the invention may be less than that required in a monotherapy utilizing only one of them as a therapeutic agent. Preferably, in these combinations or compositions, a dosage of between 0.01-1.000 µg/kg body weight/day of the one or the other therapeutic agent can be administered.

In a preferred embodiment, the degree of synergy is calculated by the Zero interaction potency (ZIP) model (Bhagwan Y. et al. 2015. Comput Struct Biotechnol J, 13: 504-513). In a more preferred embodiment, the ZIP synergy scores were calculated using SynergyFinder.org (Zheng, S.; Wang, W.; Aldahdooh, J.; Malyutina, A.; Shadbahr, T.; Tanoli, Z.; Passia, A.; Tang, J. 2022. SynergyFinder Plus: Toward Better Interpretation and Annotation of Drug Combination Screening Datasets. Genomics, Proteomics & Bioinformatics, 20(3): 587-596).

The amount of the therapeutic agents present in the combinations or compositions may be no more than the amount that would normally be administered in a composition comprising that therapeutic agent as the only active agent. Preferably, the amount of a therapeutic agent in the present compositions will range from about 50% to 100% of the amount normally present in a composition comprising that agent as the only therapeutically active agent. In some embodiments, one therapeutic agent is administered at a dosage of about 50%, about 55%, about 60%, about 65%, about 70%, about 75%, about 80%, about 85%, about 90%, or about 95% of the amount normally administered for that agent. As used herein, the phrase "normally administered" means the amount an FDA approved therapeutic agent is approved for dosing per the FDA label insert.

The combination or composition of the invention may also be used in combination with known therapeutic processes, for example, in combination with chemotherapy, radiotherapy, immunotherapy, phototherapy, surgical intervention, hormones, or a combination of these.

All the embodiments of the combination of the invention are also applicable to the therapeutic methods of the invention.

### Articles of manufacture and kits

The disclosure also provides articles of manufacture comprising any one of the combinations or the pharmaceutical compositions disclosed herein, in one or more containers. In some embodiments, the article of manufacture comprises, e.g., a brochure, printed instructions, a label, or package insert directing the user (e.g., a distributor or the final user) to combine and/or use the compositions of the article of manufacture for the prevention and/or treatment of cancer.

In some embodiments, the article of manufacture comprises, e.g., bottle(s), vial(s), cartridge(s), box(es), syringe(s), injector(s), or any combination thereof. In some embodiments, the label refers to use or administration of the combinations or the pharmaceutical compositions in the article of manufacture according to the methods disclosed herein. In some aspects, the label suggests, e.g., a regimen for use, a regimen for treating, preventing, or ameliorating a cancer.

The contents of all cited references (including literature references, patents, patent applications, and websites) that may be cited throughout this application are hereby expressly incorporated by reference in their entirety for any purpose, as are the references cited therein.

All terms as used herein, unless otherwise stated, shall be understood in their ordinary meaning as known in the art. Other more specific definitions for certain terms as used in the present application are as set forth below and are intended to apply uniformly throughout the description and claims unless an otherwise expressly set out definition provides a broader definition. Throughout the description and claims the word "comprise" and variations of the word, are not intended to exclude other technical features, additives, components, or steps. Furthermore, the word "comprise" encompasses the case of "consisting of'. Additional objects, advantages and features of the invention will become apparent to those skilled in the art upon examination of the description or may be learned by practice of the invention. Furthermore, the present invention covers all possible combinations of particular and particular embodiments described herein.

In this specification and the appended claims, the singular forms "a", "an" and "the" include plural referents unless the context clearly dictates otherwise. The terms "a" (or "an"), as well as the terms "one or more," and "at least one" can be used interchangeably herein. Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone). The term "about" as used in connection with a numerical value throughout the specification and the claims denotes an interval of accuracy, familiar and acceptable to a person skilled in the art. In general, such interval of accuracy is ± 15 %. Unless defined otherwise, all technical and scientific terms used herein have the same meaning as commonly understood by one of ordinary skill in the art to which this disclosure is related. Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various aspects or aspects of the disclosure, which can be had by reference to the specification as a whole. Accordingly, the terms defined immediately below are more fully defined by reference to the specification in its entirety.

The invention will be described by way of the following examples which are to be considered as merely illustrative and not limitative of the scope of the invention.

### EXAMPLES

### Production and purification of Omomyc

The Omomyc peptide sequence SEQ ID NO: 4 including a methionine at the N-terminal end was codon optimized for expression in *E.coli* by using "GeneGPS^{®} expression optimization technology, cloned in a medium copy number plasmids with kanamycin resistance gene as selection marker (pPC-OMO-T5) and purified from BL21 (DE3) bacterial strain using protocols adapted from the Max° purification protocol described in J.-F. Naud et al. 2003. J Mol Biol, 326:1577-1595; F.-O. and Mcduff et al. 2009. J Mol Recognit, 22:261-269. The purified construct obtained was the polypeptide of SEQ ID NO: 4. Identity of each purified construct was confirmed by mass spectrometry and by western blot analysis. Omomyc was purified by cationic exchange chromatography and purity was confirmed by mass spectrometry analysis, SDS-PAGE and UV spectroscopy.

### Experimental details

Several KRAS mutant cell lines were used to test the synergy of Omomyc (MYC inhibitor) with different KRAS inhibitors. The list of the cell lines used and their KRAS mutation is shown in **Table 2.** HCC44, H1792, H23, H358, and SW1463 are commercial cell lines. KLA-p53 and KLA-Stk11 are KRAS Lung Adenocarcinoma (KLA) cell lines derived from the transgenic KRAS^{G12D}-driven Non-Small Cell Lung Cancer (NSCLC) mouse model and are manipulated by the CRISPR/Cas9 technology to obtain the specific knockout of TP53 (KLA-p53) and STK11 (KLA-Stk11) genes. Lim1215 is a commercially available Colorectal Cancer (CRC) cell line (originally KRAS wild type) that has been genetically modified to express the KRAS G12D mutation using the CRISPR/Cas9 technology.

**Table 1. KRAS mutant cell lines. NSCLC: Non-Small Cell Lung Cancer. CRC: colorectal cancer.**

| **Cell Line** | **Cancer Type** | **KRAS mutation** |
|---|---|---|
| HCC44 | NSCLC | G12C |
| H1792 | NSCLC | G12C |
| H23 | NSCLC | G12C |
| H358 | NSCLC | G12C |
| SW1463 | CRC | G12C |
| KLA-p53 | NSCLC | G12D |
| KLA-Stk11 | NSCLC | G12D |
| Lim1215 - KRASG12D | CRC | G12D |

To assess the potential synergy of Omomyc and different KRAS inhibitors, cells were seeded in 96 well plates. 24h after seeding, cells were treated with increasing concentrations of Omomyc (such as 1.25, 2.5, 5, 10 and 20 µM) and increasing concentrations of the different KRAS inhibitors. After 5 days, AlamarBlue (metabolic assay to indirectly measure the viable cells) was added to the wells and 4-8 hours later fluorescence at 590 nm was measured with a Spark microplate reader. To determine if the effect of the combination of the two drugs was synergistic, ZIP synergy scores were calculated and plotted using SynergyFinder.org (Zheng, S.; Wang, W.; Aldahdooh, J.; Malyutina, A.; Shadbahr, T.; Tanoli, Z.; Passia, A.; Tang, J. 2022. SynergyFinder Plus: Toward Better Interpretation and Annotation of Drug Combination Screening Datasets. Genomics, Proteomics & Bioinformatics, 20(3): 587-596). When the synergy score is ≤10, the interaction between two drugs is considered to be antagonistic; from -10 to 10, it is considered to be additive; and when >10, it is considered to be synergistic (Yadav B, Wennerberg K, Aittokallio T, Tang J. 2015. Searching for drug synergy in complex dose-response landscapes using an interaction potency model. Comput Struct Biotechnol J, 13: 504-13). Statistical analysis was performed using the Graph-Pad Prism 8. To determine statistical significance between the combination treatment and the monotherapies alone, a one-way ANOVA with Dunnett's post hoc test was performed.

### Results

In order to determine if Omomyc was able to synergize with KRAS G12C inhibitors, KRASG12C-mutant NSCLC and CRC cell lines (HCC44, H1792, H23, H358 and SW1463) were treated with increasing concentrations of Omomyc and Sotorasib (KRAS G12C inhibitor). Cell viability was assessed using the AlamarBlue metabolic assay. In all cell lines, the combination of Omomyc and Sotorasib was significantly more potent than either stand-alone therapy and showed synergy in many different drug concentrations. Synergy at representative concentrations is shown in **Figure 1****.**

The synergistic effect of the combination of Omomyc with another KRASG12C inhibitor, Adagrasib, was also investigated. Again, in KRASG12C-mutant NSCLC and CRC cell lines (HCC44, H1792, H23, H358 and SW1463), the combination of Omomyc and Adagrasib was significantly more potent than both monotherapies and showed synergy in many different drug concentrations. Synergy at representative concentrations is shown in **Figure 2****.**

In order to determine if Omomyc was able to synergize with KRASG12D inhibitors too, KRASG12D-mutant NSCLC and CRC cell lines (KLA-p53, KLA-Stk11 and Lim1215) were treated with the combination of Omomyc and MRTX1133 (KRAS G12D inhibitor). Again, the combination of Omomyc and MRTX1133 was significantly superior to both monotherapies and showed synergy in many different drug concentrations. Synergy at representative concentrations is shown in **Figure 3****.**

The authors investigated also the synergistic effect of Omomyc with pan-KRAS inhibitors. An example of these pan-KRAS inhibitors are the inhibitors that target the SOS1 molecule, which is upstream of KRAS and is responsible for KRAS activation. In this regard, the synergistic effect of the combination of Omomyc with two pan-KRAS inhibitors, BAY-293 and BI-3406, was tested. In order to determine if Omomyc synergizes with BAY-293, H358, HCC44, KLA-p53 and SW1463 KRAS mutant cell lines were treated with increasing concentrations of Omomyc and BAY-293. In order to determine if Omomyc synergizes with BI-3406, H358, HCC44 and KLA-p53 KRAS mutant cell lines were treated with increasing concentrations of Omomyc and BI-3406. Results show that, once more, Omomyc is able to synergize with both BAY-293 (**Figure 4**) and BI-3406 (**Figure 5**) and that the combination is superior to standalone therapies and showed synergy in many different drug concentrations.

Taken all these results together, it clearly indicates that Omomyc efficiently synergizes with both direct and indirect KRAS inhibitors in many different concentration ranges and in different cancer types. Combination of Omomyc with KRAS inhibitors represent effective combination partners that provide an attractive therapeutic concept for the majority of, if not all, KRAS-mutant cancers, regardless of their specific KRAS mutation.

## Claims

1. A combination comprising:
i) a first component selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
and
ii) a second component that is a KRAS inhibitor.

2. The combination according to claim 1, wherein the first component is a polypeptide comprising the sequence SEQ ID NO: 1.

3. The combination according to claim 1, wherein the functionally equivalent variant of SEQ ID NO: 1 is selected from the group consisting of SEQ ID NO: 4, SEQ ID NO: 5, SEQ ID NO; 6, SEQ ID NO: 7, SEQ ID NO: 8, SEQ ID NO: 9, and SEQ ID NO: 10.

4. The combination according to any one of claims 1 or 3, wherein the chemical moiety that facilitates the cellular uptake of the polypeptide or of the functionally equivalent variant thereof is a cell-penetrating peptide sequence and wherein said cell penetrating peptide sequence and said polypeptide or functionally equivalent variant thereof form a fusion protein.

5. The combination according to any one of claims 1 to 4, wherein the KRAS inhibitor is selected from the group consisting of a KRAS G12C inhibitor, a KRAS G12D inhibitor, and a pan-KRAS (SOS1) inhibitor; more preferably is selected from a KRAS G12C inhibitor and a KRAS G12D inhibitor.

6. The combination according to claim 5, wherein the KRAS inhibitor is selected from the group consisting of sotorasib, adagrasib and MRTX1133.

7. The combination according to claim 5, wherein the KRAS inhibitor is a pan-KRAS (SOS1) inhibitor.

8. The combination according to claim 7, wherein the pan-KRAS (SOS1) inhibitor is selected from the group consisting of BAY-293 and BI-3406.

9. A pharmaceutical composition comprising a pharmaceutically effective amount of a combination according to any one of claims 1 to 8 and a pharmaceutically acceptable excipient.

10. A combination according to any one of claims 1 to 8 or a pharmaceutical composition according to claim 9 for use in medicine.

11. A combination according to any one of claims 1 to 8 or a pharmaceutical composition according to claim 9 for use in the prevention and/or treatment of cancer.

12. The combination or pharmaceutical composition for use according to claim 11, wherein the cancer is selected from the group consisting of NSCLC and colorectal cancer

13. The combination or pharmaceutical composition for use according to any one of claims 11 or 12, wherein the cancer is a KRAS mutated cancer.

14. The combination or pharmaceutical composition for use according to any one of claims 11 to 13, wherein the cancer is a cancer resistant to KRAS inhibitors.

15. The combination or pharmaceutical composition for use according to any one of claims 10 to 14, wherein the first component is administered intravenously and the second component is administered orally.
